# EUROPEAN PATENT APPLICATION

(11) **EP 4 541 366 A1**
(43) Date of publication of application: **23.04.2025**
(21) Application number: 23824239.0
(22) Date of filing: 15.06.2023
(51) Int. Cl.: A61K 38/12, A61K 31/426, A61K 39/395, A61K 45/06, A61P 35/00, C07K 16/28, A61K 39/00

(54) **PHARMACEUTICAL COMPOSITION COMPRISING FOXM1 INHIBITOR AND IMMUNE CHECKPOINT INHIBITOR FOR PREVENTING OR TREATING CANCER**

(30) Priority: 16.06.2022 KR 20220073323; 08.09.2022 KR 20220114268
(71) Applicant: National Cancer Center, Goyang-si, Gyeonggi-do 10408 (KR)
(72) Inventor: CHOI, Yong Doo, Goyang-si Gyeonggi-do 10408 (KR); GOH, Sung Ho, Goyang-si Gyeonggi-do 10416 (KR)
(74) Representative: Algemeen Octrooi- en Merkenbureau B.V.
(86) International application number: PCT/KR2023/008264
(87) International publication number: WO 2023/244026

(57) **Abstract**

The present invention relates to a pharmaceutical composition for preventing, ameliorating, or treating cancer, the pharmaceutical composition comprising an FOXM1 inhibitor and an immune checkpoint inhibitor as active ingredients. The FOXM1 inhibitor of the present invention inhibits the expression of PD-L1 in a cell membrane and inhibits the translocation of FOXM1 to the nucleus, thereby reducing the proliferation and survival of cancer cells and inducing an increase in cell death, and thus has the effect of preventing, ameliorating, or treating cancer. In addition, when the FOXM1 inhibitor is administered in combination with an immune checkpoint inhibitor, tumor growth is inhibited more effectively than when the FOXM1 inhibitor or immune checkpoint inhibitor is used alone, and thus the pharmaceutical composition according to the present invention can be useful as an agent for preventing or treating cancer.

## Description

### [Technical Field]

The present disclosure relates to a pharmaceutical composition for preventing or treating cancer, comprising a FOXM1 inhibitor and an immune checkpoint inhibitor as active ingredients.

### [Background Art]

Lung cancer is the second most common cancer in the world after breast cancer, and despite significant advances in cancer treatment technology, it still has the highest mortality rate of all cancers. Non-small cell lung cancer (NSCLC) accounts for 80 to 85% of all lung cancer patients, and while immune cells infiltrate many NSCLC tissues, cytotoxic T lymphocytes in tumor tissues are known to be functionally inhibited by various mechanisms, including abnormal overexpression of immune checkpoint proteins utilized by tumor cells.

Immune checkpoint inhibitors (ICIs), which are a type of cancer immunotherapy, are drugs that block proteins called immune checkpoints expressed on immune cells, such as T cells, and cancer cells. Immunotherapy using immune checkpoint inhibitors has recently significantly improved therapeutic effect in various carcinomas, including lung cancer. Programmed cell death-ligand-1 (PD-L1) is encoded by CD274 gene and is an important immune checkpoint molecule that mediates the interaction between cancer cells and tumor-infiltrating T cells. Programmed cell death-1 (PD-1) is expressed on a surface of T cells, and PD-L1 is expressed on normal tissues to inhibit self-aggression by immune system. However, PD-L1 overexpressed on the surface of cancer cells allows cancer cells to evade attacks by immune cells. Therefore, ICIs, such as anti-PD-1 or anti-PD-L1 antibodies, which inhibit the interaction between PD-1 and PD-L1, can enhance the therapeutic effect of cancers, such as lung cancer by reactivating the attack of cancer cells by immune cells. However, it has been reported that expensive ICI treatment not only places a significant financial burden on cancer patients, but also has various immune-related side effects, including cardiac complications, in non-target organs. In addition, it has been reported that the therapeutic effect of ICI is not significant in case of large tumor size or rapid tumor growth. Therefore, it is necessary to develop new strategies or treatments that can solve these problems of ICIs.

Forkhead box protein M1 (FOXM1) is a protein that is important in regulating various processes related to lung cancer tumorigenesis, including cell cycle progression, chemotherapy resistance, and metastasis. The function of FOXM1 is important for the survival of cancer cells, in part due to its ability of FOXM1 to translocate to the nucleus through various forkhead or DNA-binding domains and bind to regulatory regions of multiple target genes. Compounds such as thiostrepton (TST), siomycin A, Robert Costa Memorial drug-1 (RCM-1), and forkhead domain inhibitor-6 (FDI-6) have been identified as FOXM1 inhibitors. Among these, TST, which is a natural thiazole-based antibiotic isolated from *Streptomyces azureus,* inhibits the interaction of the forkhead domain of FOXM1 with target DNA. In addition, TST has been used to treat mastitis caused by gram-negative bacteria and has been reported to be active against breast cancer.

Against this background, the present inventors confirmed that a FOXM1 inhibitor significantly reduces PD-L1 expression and inhibits proliferation of tumor cells through research using the FOXM1 inhibitor and thus has the effect of preventing or treating cancer, thereby completing the present disclosure.

### [Disclosure]

### [Technical Problem]

An object of the present disclosure is to provide a pharmaceutical composition for preventing or treating cancer, comprising a FOXM1 inhibitor and an immune checkpoint inhibitor as active ingredients.

Another object of the present disclosure is to provide an anticancer adjuvant comprising a FOXM1 inhibitor and an immune checkpoint inhibitor as active ingredients.

Another object of the present disclosure is to provide use of a composition comprising a FOXM1 inhibitor and an immune checkpoint inhibitor for the prevention or treatment of cancer.

Another object of the present disclosure is to provide use of a composition comprising a FOXM1 inhibitor and an immune checkpoint inhibitor for the manufacture of a medicament for the prevention or treatment of cancer.

Another object of the present disclosure is to provide a method for preventing or treating cancer, comprising administering to a subject a composition comprising a FOXM1 inhibitor and an immune checkpoint inhibitor.

### [Technical Solution]

This will be specifically described as follows. Meanwhile, each of the descriptions and embodiments disclosed in the present application may also be applied to each other descriptions and embodiments. That is, all combinations of various elements disclosed in the present application fall within the scope of the present application. In addition, the scope of the present application is not to be considered limited by the specific description set forth below.

As an aspect to achieve the above objects, the present disclosure provides a pharmaceutical composition for preventing or treating cancer, comprising a FOXM1 inhibitor and an immune checkpoint inhibitor as active ingredients.

As used herein, the "forkhead box protein M1 (FOXM1)" is a protein belonging to a FOX family of transcription factors, is encoded by FOXM1 gene, and is known to play an important role in cell cycle progression.

As used herein, the "FOXM1 inhibitor" is a substance that inhibits FOXM1, and may be any substance that inhibits or reduces the expression of FOXM1. Specifically, the FOXM1 inhibitor may be thiazolidinedione, diarylheptanoid, RCM-1, thiostrepton, honokiol, FDI-6, Siomycin A, monensin, FOXM1 aptamer (FOXM1 apt), peptide 9R-P201, or a pharmaceutically acceptable salt thereof.

The thiazolidinedione-based compound is a type of heterocyclic compound composed of a five-membered C₃NS ring, which has been reported as pharmaceuticals that can be used to treat type 2 diabetes by lowering blood sugar levels, and may be, for example, TFI-1 to TFI-10, preferably TFI-10, but is not limited thereto.

The compound TFI-10 is a thiazolidinedione forkhead domain inhibitor, and refers to a substance having a structure of Formula 1 of (5Z)-5-[(3-methoxy-4-{[4-(trifluoromethyl)phenoxy]methyl}phenyl)methylidene]-1,3-thiazolidine-2,4-dione as a skeleton:

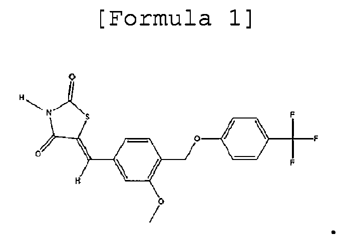

The diarylheptanoid refers collectively to secondary metabolites of plants composed of two aromatic rings (aryl groups) connected by a seven-carbon chain (heptane) and having various substituents, and is known to have antioxidant activity.

The RCM-1 is a FOXM1 inhibitor known to block nuclear localization and increase proteasomal degradation of FOXM1, and refers to a substance having a structure of Formula 2:

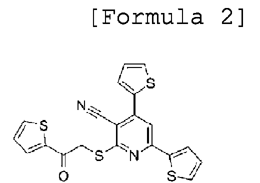

The thiostrepton is a natural cyclic oligopeptide antibiotic of the thiopeptide family derived from several Streptomyces strains such as *Streptomyces azureus* and *Streptomyces laurentii,* and refers to a substance having a structure of Formula 3:

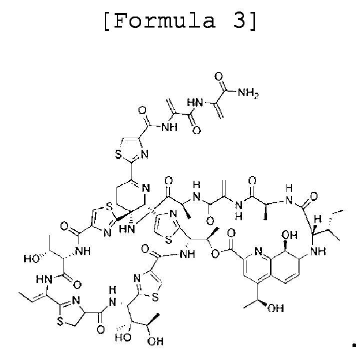

The honokiol is a lignan isolated from the bark, seeds, and leaves of a tree belonging to the genus Magnolia, is known to have high bioavailability because it can easily pass through a blood-brain barrier and a blood-cerebrospinal fluid barrier, and refers to a substance having a structure of Formula 4:

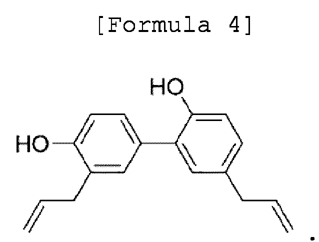

The FDI-6 is a FOXM1 inhibitor that directly binds to the FOXM1 protein and induces transcriptional downregulation, and refers to a substance having a structure of Formula 5:

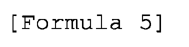

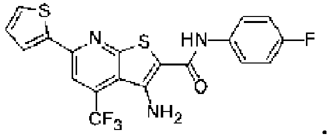

The Siomycin A is a thiazole-based antibiotic that has been reported to downregulate mRNA and protein expression as well as the transcriptional activity of FOXM1, and refers to a substance having a structure of Formula 6:

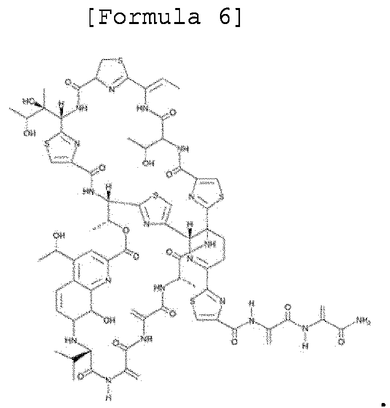

The monensin, also called monensin, monensin A, monensina, monensinum, or rumensin, is a polyether antibiotic isolated from *Streptomyces cinnamonensis,* and refers to a substance having a structure of Formula 7:

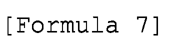

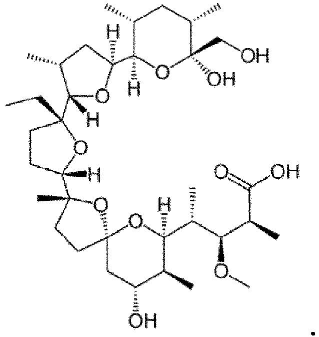

The FOXM1 aptamer (FOXM1 apt) refers collectively to a FOXM1-specific aptamer that targets a FOXM1 DNA binding domain, and may comprise, for example, but is not limited to, a sequence of SEQ ID NO: 9.

The peptide 9R-P201 is a peptide that has been reported to inhibit the survival, proliferation, and migration of cancer cells and induce apoptotic cell death by downregulating FOXM1 expression, and refers to a peptide substance (Jian Cui et al. Int J Pept Res Ther 20, 447-456(2014)) having nine arginines conjugated to P201 (peptide).

The FOXM1 inhibitor of the present disclosure may, for example, reduce gene or protein levels of FOXM1, or reduce the translocation of FOXM1 to the nucleus.

As used herein, the FOXM1 inhibitor may be obtained from biological sources known in the art, or may be chemically synthesized or commercially available.

As used herein, the FOXM1 inhibitor comprises a form of a pharmaceutically or food-acceptable salt to the extent that thy have the same efficacy. Such salt may be a pharmaceutically or food-wise acceptable salt, and may be a basic salt or an acidic salt, wherein the basic salt may be in the form of either an organic basic salt or an inorganic basic salt, and may be selected from the group consisting of a sodium salt, a potassium salt, a calcium salt, a lithium salt, a magnesium salt, a cesium salt, an aluminum salt, an ammonium salt, a triethylaminium salt, and a pyridinium salt.

Among these types of salts, as acidic salts, acid addition salts formed by free acids are useful. As free acids, inorganic acids and organic acids may be used. As inorganic acids, hydrochloric acid, hydrobromic acid, sulfuric acid, sulfurous acid, phosphoric acid, diphosphoric acid, nitric acid, etc. may be used. As the organic acids, citric acid, acetic acid, maleic acid, malic acid, fumaric acid, gluconic acid, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, aspartic acid, stearic acid, etc. may be used, but are not limited thereto. All salts formed using various inorganic acids and organic acids commonly used in the art may be included.

As used herein, the "immune checkpoint inhibitors" are a form of cancer immunotherapy and refer to drugs that block proteins called immune checkpoints expressed on some immune cells, such as T cells, and cancer cells, and may specifically be, but is not limited to, a PD-L1 inhibitor, an anti-PD-L1 antibody, a PD-1 inhibitor, an anti-PD-1 antibody, a CTLA inhibitor, an anti-CTLA4 antibody, an anti-PD-L2 antibody, an LTF2 regulatory antibody, an anti-LAG3 antibody, an anti-A2aR antibody, an anti-TIGIT antibody, an anti-TIM-3 antibody, an anti-B7-H3 antibody, an anti-B7-H4 antibody, an anti-VISTA antibody, an anti-CD47 antibody, an anti-BTLA antibody, an anti-KIR antibody, an anti-IDO antibody, or an anti-4-1BB antibody.

As used herein, the "active ingredient" refers to an ingredient capable of exhibiting desired activity alone or in combination with a carrier that is itself inactive.

As used herein, the "cancer (or tumor)" may be solid cancer or blood cancer, regardless of whether it is primary cancer or metastatic cancer. Specifically, the cancer may be, but is not limited to, lung cancer, pancreatic cancer, pharyngeal cancer, laryngeal cancer, esophageal cancer, stomach cancer, duodenal cancer, small intestine cancer, colorectal cancer, colon cancer, rectal cancer, breast cancer, ovarian cancer, prostate cancer, liver cancer, thyroid cancer, kidney cancer, uterine cancer, brain tumor, skin cancer, melanoma, malignant bone tumor, bladder cancer, or blood cancer. More specifically, the cancer may be lung cancer or colorectal cancer.

As used herein, the term "prevention" refers to any act inhibiting or delaying the development of cancer by administration of the composition according to the present disclosure, the term "amelioration" refers the effect of delaying the progression of cancer or alleviating the symptoms of cancer by applying the composition according to the present disclosure, and the term "treatment" refers to any act that is beneficially changed, such as completely inhibiting the progression of cancer or improving the symptoms of cancer, by administering the composition according to the present disclosure.

The composition according to the present disclosure has the effect of preventing, ameliorating, or treating cancer by, for example, reducing or inhibiting the expression of PD-L1 to reduce the proliferation and survival of cancer cells and induce an increase in apoptotic cell death.

A decrease in the expression of PD-L1 may mean a decrease in the expression level of PD-L1 protein in the cell membrane.

As an aspect for achieving the above objects, if the FOXM1 inhibitor of the present disclosure is a hydrophobic compound with low solubility in water, it may be provided in a form of being loaded into a liposome as a system for effectively delivering the hydrophobic compound to a tumor, and specifically, it may be loaded into the surface, interior, or phospholipid bilayer of the liposome, but is not limited thereto.

The liposome may refer to a bilayer membrane structure composed of phospholipids, and preferably a micelle structure.

The phospholipids constituting the liposome may be at least one selected from, but are not necessarily limited to, the group consisting of 1,2-dioleoyl-sn-glycero-2-phosphocholine (DOPC), 1,2-distearoyl-sn-glycero-3-phosphoethanolamine-N-[methoxy(polyethylene glycol)-2000] (DSPE-PEG2000), cholesterol, 1,2-distearoyl-sn-glycero-3-phosphocholine, 1,2-distearoyl-sn-glycero-3-phosphoethanolamine-N-[methyl(polyethylene glycol)-5000], 1,2-distearoyl-sn-glycero-3-phosphoethanolamine-N-[methyl(polyethylene glycol)-5000] ammonium salt, dioleoyl phosphatidylcholine, 1,2-dioleoyl-sn-glycero-3-phosphoethanolamine, dipalmitoylphosphatidylcholine, and cholesteryl hemisuccinate.

There is no particular limitation on a content ratio of the phospholipids constituting the liposome, and the ratio may be set freely, taking into account the size of the liposome to be prepared.

As an aspect for achieving the above objects, the liposome of the present disclosure may be composed of DOPC, cholesterol, and DSPE-PEG2000, and specifically, a molar ratio of each component may be, but is not limited to, 5 to 15, 3 to 13, and 1 to 12.

The pharmaceutical composition according to the present disclosure may be administered in combination with a FOXM1 inhibitor and an immune checkpoint inhibitor. Specifically, the pharmaceutical composition may be in the form of a mixture of a FOXM1 inhibitor and an immune checkpoint inhibitor, or may be formulated with each of the FOXM1 inhibitor and the immune checkpoint inhibitor and administered simultaneously or sequentially, but is not limited thereto.

When a FOXM1 inhibitor and an immune checkpoint inhibitor are administered in combination, a ratio of the administration concentration of the FOXM1 inhibitor and the immune checkpoint inhibitor may be 1:0.01 to 1:1000, specifically 1:0.01 to 1:500, 1:0.05 to 1:300, 1:0.1 to 1:200, or 1:1 to 1:100, and more specifically 1:0.01, 1:0.02, 1:0.03, 1:0.05, 1:0.08, 1:0.1, 1:0.3, 1:0.5, 1:0.7, 1:0.8, 1:1, 1:1.5, 1:2, 1:3, 1:5, 1:7, 1:8, 1:10, 1:25, It can be, but is not limited to, 1:50, 1:100, 1:200, or 1:500, but is not limited thereto.

When a FOXM1 inhibitor and an immune checkpoint inhibitor loaded into a liposome are administered in combination, a ratio of the administration concentration of the FOXM1 inhibitor and the immune checkpoint inhibitor loaded into the liposome may be 1:0.01 to 1:1000, specifically 1:0.01 to 1:500, 1:0.05 to 1:300, 1:0.1 to 1:200 or 1:1 to 1:100, and more specifically 1:0.01, 1:0.02, 1:0.03, 1:0.05, 1:0.08, 1:0.1, 1:0.3, 1:0.5, 1:0.7, 1:0.8, 1:1, 1:1.5, 1:2, 1:3, 1:5, 1:7, 1:8, 1:10, 1:25, 1:50, 1:100, 1:200, or 1:500, but is not limited thereto.

The pharmaceutical composition according to the present disclosure may further comprise pharmaceutically acceptable carriers, excipients, or diluents according to conventional methods. The pharmaceutically acceptable carriers are known in the art, depending on the route of administration or formulation, and may refer specifically to the pharmacopoeias of each country, including the "Korean Pharmacopoeia." Examples of carriers, excipients, and diluents that may be included in the composition according to the present disclosure include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. In addition, carriers, excipients, and diluents that may be included in the compositions according to the present disclosure may be non-natural carriers, but are not limited thereto.

The pharmaceutical composition according to the present disclosure may be formulated and used according to conventional methods in the form of oral formulations such as powders, granules, tablets, capsules, suspensions, emulsions, syrups, aerosols, external preparations, suppositories, or sterile injection solutions. Specifically, when the pharmaceutical composition is formulated, it may be prepared using commonly used diluents or excipients such as fillers, extenders, binders, wetting agents, disintegrants, and surfactants. Solid preparations for oral administration include tablets, pills, powders, granules, capsules, etc., and may be prepared by mixing the compound with at least one excipient, for example, starch, calcium carbonate, sucrose, lactose, gelatin, etc. Further, in addition to simple excipients, lubricants such as magnesium stearate and talc may also be used. Liquid preparations for oral administration include suspensions, solutions, emulsions, and syrups, and various excipients, for example, wetting agents, sweeteners, fragrances, preservatives, etc., in addition to water and liquid paraffin, which are commonly used simple diluents. Preparations for parenteral administration include sterile aqueous solutions, non-aqueous solutions, suspensions, emulsions, lyophilized preparations, and suppositories. As non-aqueous solutions and suspensions, propylene glycol, polyethylene glycol, vegetable oils such as olive oil, and injectable esters such as ethyl oleate may be used. As a base for a suppository, Witepsol, macrogol, Tween 61, cacao butter, laurin butter, glycerogelatin, etc. may be used. Specific formulations of the pharmaceutical compositions are known in the art, and can be found in, for example, Remington's Pharmaceutical Sciences (19th ed. 1995). The reference is hereby considered to be a part of the present specification.

The pharmaceutical composition according to the present disclosure is administered in a pharmaceutically effective amount. The pharmaceutically effective amount refers to an amount that is sufficient to treat the disease at a reasonable benefit/risk ratio applicable for any medical treatment, and is unlikely to cause side effects. The effective dose level may be determined depending on factors including patient's health condition, type and severity of the disease, activity of the drug, sensitivity to the drug, administration method, administration time, administration route, and excretion rate, duration of treatment, combination, or concomitant medications, and other factors well known in the medical field. The dosage and frequency of administration are not intended to limit the scope of the present disclosure in any way.

The pharmaceutical composition according to the present disclosure may be administered to mammals such as mice, dogs, cats, cows, horses, pigs, and humans through various routes.

The pharmaceutical composition according to the present disclosure may be applied in any formulation comprising a FOXM1 inhibitor and an immune checkpoint inhibitor as active ingredients, and may be prepared as an oral or parenteral formulation. Specifically, the mode of administration is not limited, but include forms suitable for oral, rectal, nasal, topical (including buccal and sublingual), subcutaneous, vaginal, or parenteral (including intramuscular, subcutaneous, and intravenous) administration, or forms suitable for administration by inhalation or insufflation.

As an aspect for achieving the above objects, the present disclosure provides an anticancer adjuvant comprising a FOXM1 inhibitor and an immune checkpoint inhibitor as active ingredients.

The "FOXM1 inhibitor", "immune checkpoint inhibitor", "active ingredient", and "cancer" are as described above.

The anticancer adjuvant according to the present disclosure refers to any form for enhancing the anticancer effect of an anticancer drugs or inhibiting or improving the side effects of an anticancer drug. The anticancer adjuvant according to the present disclosure may be administered in combination with various types of anticancer drugs or anticancer adjuvants. When administered in combination, even if the anticancer drug is administered at a lower level than the usual dose of an anticancer drug, the same level of anticancer therapeutic effect may be achieved, such that safer anticancer treatment may be performed.

The anticancer adjuvant may be administered through any general route as long as it can reach the target tissue. The anticancer adjuvant according to the present disclosure may be suitable for oral, rectal, nasal, topical (including buccal and sublingual), subcutaneous, vaginal, or parenteral (including intramuscular, subcutaneous and intravenous) administration, or may be administered by inhalation or insufflation, depending on the intended use, but is not limited thereto. In addition, the anticancer adjuvant may be administered by any device capable of transporting the active substance to the target cell.

The anticancer adjuvant according to the present disclosure may be preferably formulated as an anticancer adjuvant by additionally including one or more pharmaceutically acceptable carriers in addition to the effective ingredient for administration. The carriers, excipients or diluents that may be included in the anticancer therapeutic adjuvant according to the present disclosure include, but are not limited to, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In an aspect for achieving the above objects, the present disclosure provides use of a composition comprising a FOXM1 inhibitor and an immune checkpoint inhibitor, for the prevention or treatment of cancer.

The "cancer", "prevention", "treatment", "FOXM1 inhibitor", "immune checkpoint inhibitor", and "composition" are as described above.

In an aspect for achieving the above objects, the present disclosure provides a use of a composition comprising a FOXM1 inhibitor and an immune checkpoint inhibitor for the manufacture of a medicament for the prevention or treatment of cancer.

The "cancer", "prevention", "treatment", "FOXM1 inhibitor", "immune checkpoint inhibitor", and "composition" are as described above.

In an embodiment for achieving the above objects, the present disclosure provides a method for preventing or treating cancer comprising administering to a subject a composition comprising a FOXM1 inhibitor and an immune checkpoint inhibitor.

The "cancer", "prevention", "treatment", "FOXM1 inhibitor", "immune checkpoint inhibitor", and "composition" are as described above.

### [Advantageous Effects]

The pharmaceutical composition comprising a FOXM1 inhibitor and an immune checkpoint inhibitor according to the present disclosure inhibits tumor growth and induce cell death more effectively than the FOXM1 inhibitor or the immune checkpoint inhibitor alone to exhibit the effect of preventing, ameliorating, or treating cancer, and thus may be useful as a preventive or therapeutic agent for these cancers.

### [Description of Drawings]

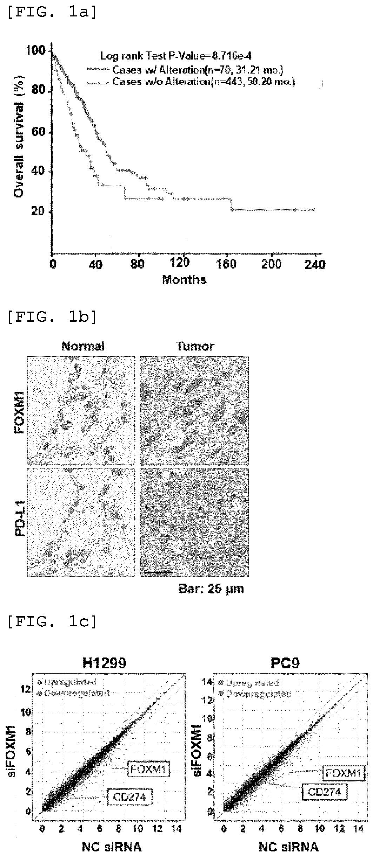

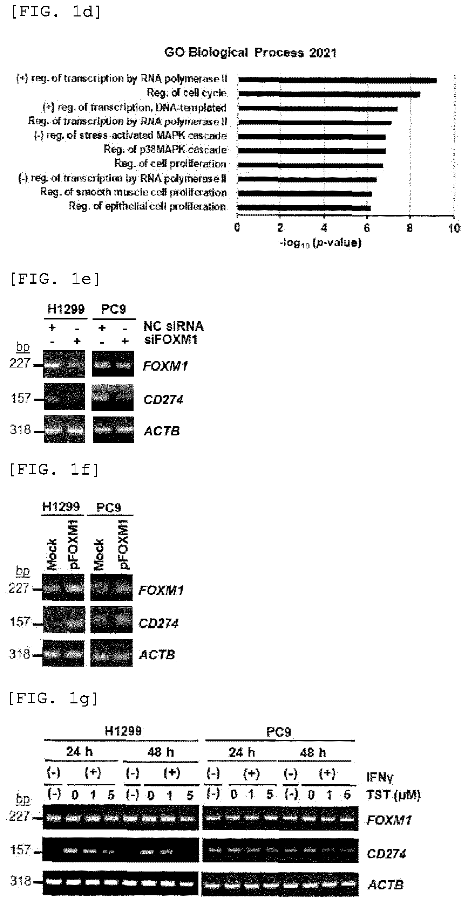

FIG. 1 shows the results confirming that PD-L1 is downregulated in a FOXM1-knockdown lung adenocarcinoma (LUAD) cell line. FIG. 1a shows the overall survival curves for lung adenocarcinoma patients with (red line) or without (blue line) simultaneous alteration in the expression of FOXM1 and PD-L1. FIG. 1b shows the results of immunohistochemical analysis of the expression levels of FOXM1 and PD-L1 in human lung cancer tissues and normal lung tissues. FIGS. 1c and 1d show the results of performing RNA-seq on H1299 and PC9 cells transfected with siFOXM1, followed by gene ontology functional activation analysis. FIGS. 1e, 1f, and 1g show the results confirming the expression levels of FOXM1, CD274 (PD-L1) following FOXM1 knockdown, overexpression, or thiostrepton (TST) treatment in H1299 and PC9 cells by semi-quantitative PCR.

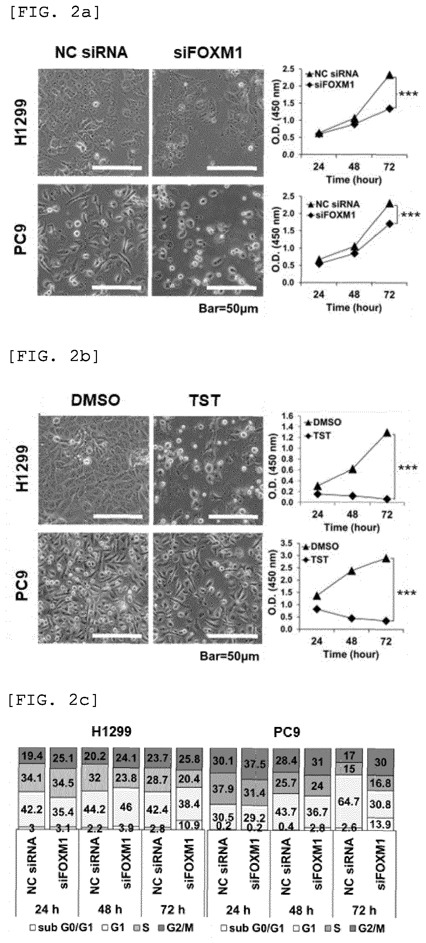

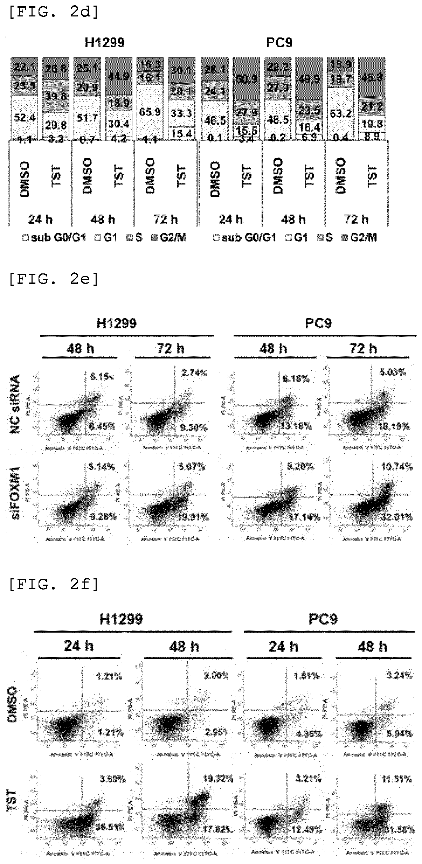

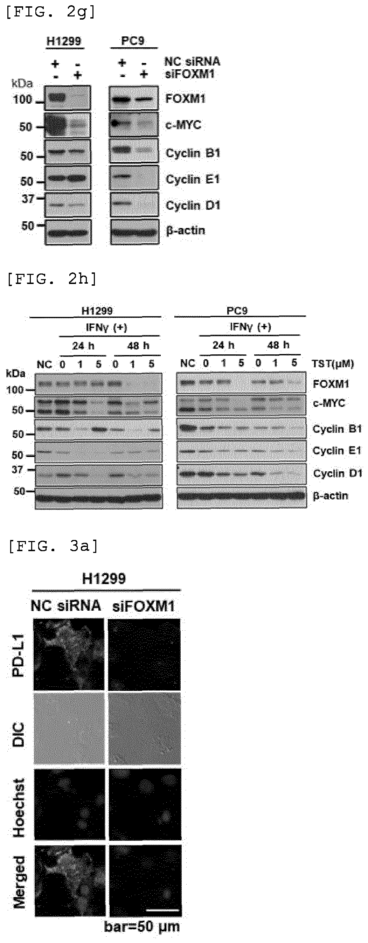

FIG. 2 shows the results confirming the association of thiostrepton (TST)-mediated downregulation of FOXM1 with decreased cell proliferation, decreased survival, and increased apoptotic cell death. The apoptotic effect upon FOXM1 depletion by transfection of siFOXM1 or treatment with thiostrepton (5 µM) is confirmed (FIGS. 2a and 2b), the results of FACS analysis of DNA content by PI staining are shown (FIGS. 2c and 2d), and the results of FACS analysis of apoptotic cells after staining with Annexin V and PI are shown (FIGS. 2e and 2f). FIGS. 2g and 2h show the results confirming the protein expression levels of FOXM1, c-MYC, cyclin B1, cyclin E1, and cyclin D1 in cells treated with siFOXM1 knockdown or thiostrepton.

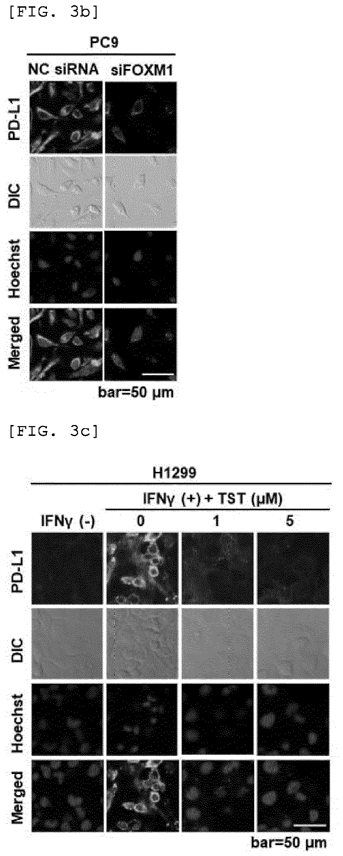

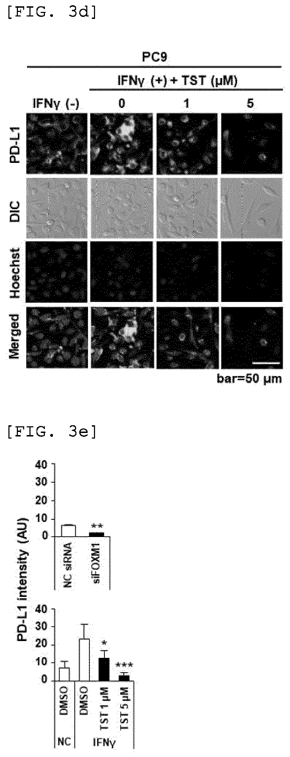

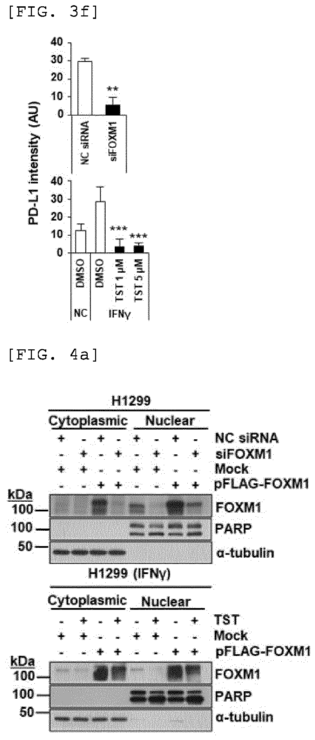

FIG. 3 shows the results confirming that the downregulation of FOXM1 by thiostrepton (TST) is associated with reduced PD-L1 expression on the cell membrane. Fluorescence imaging results are shown for H1299 cells (FIGS. 3a to 3c) and PC9 cells (FIGS. 3b to 3d) treated with siFOXM1 knockdown or thiostrepton. FIGS. 3e and 3f show the results of quantitative analysis of PD-L1 fluorescence signals in the fluorescence images.

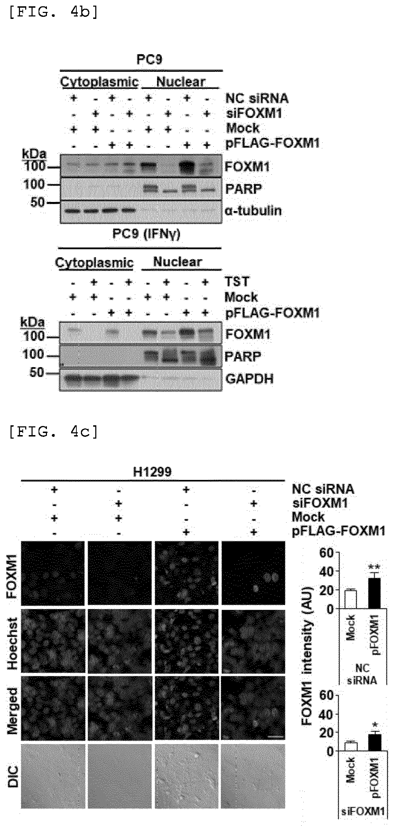

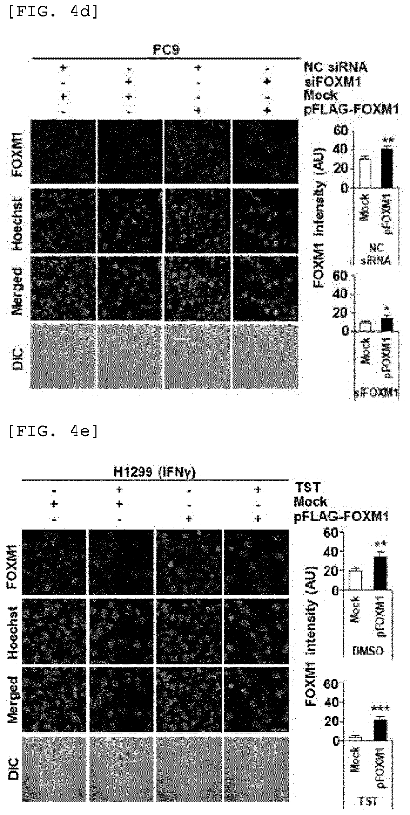

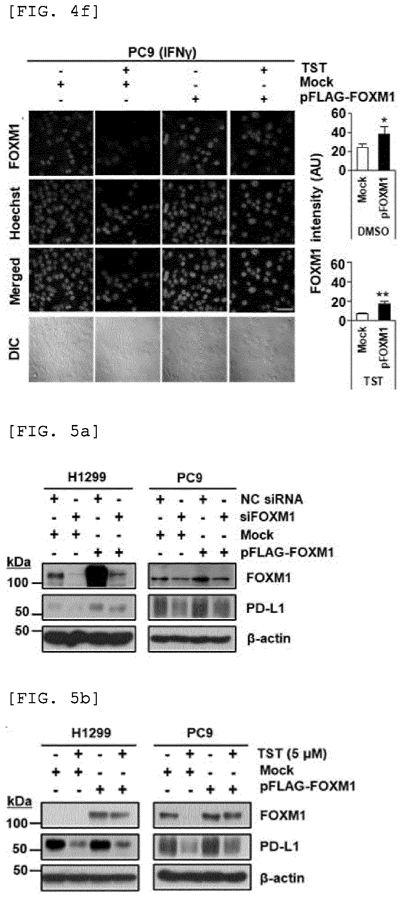

FIG. 4 shows the results confirming that the translocation of FOXM1 to the nucleus is inhibited by thiostrepton (TST) treatment. The changes in the protein levels of FOXM1 in the cytoplasmic and nuclear fractions of FOXM1 knockdown, overexpression, or TST-treated H1299 cells and PC9 cells were evaluated by Western blotting (FIGS. 4a and 4b) and fluorescence imaging (FIGS. 4c to 4f).

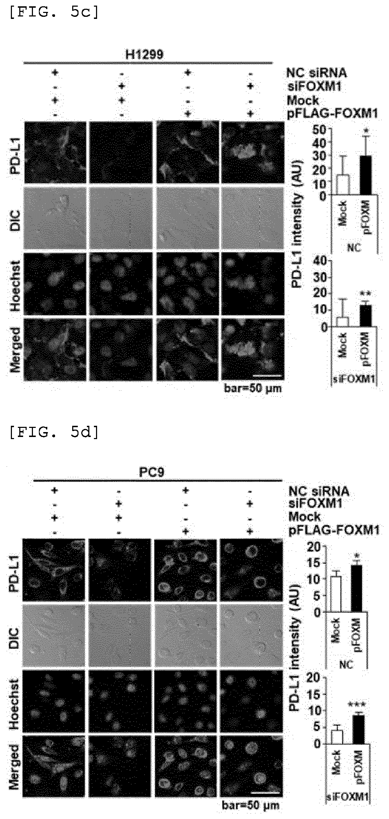

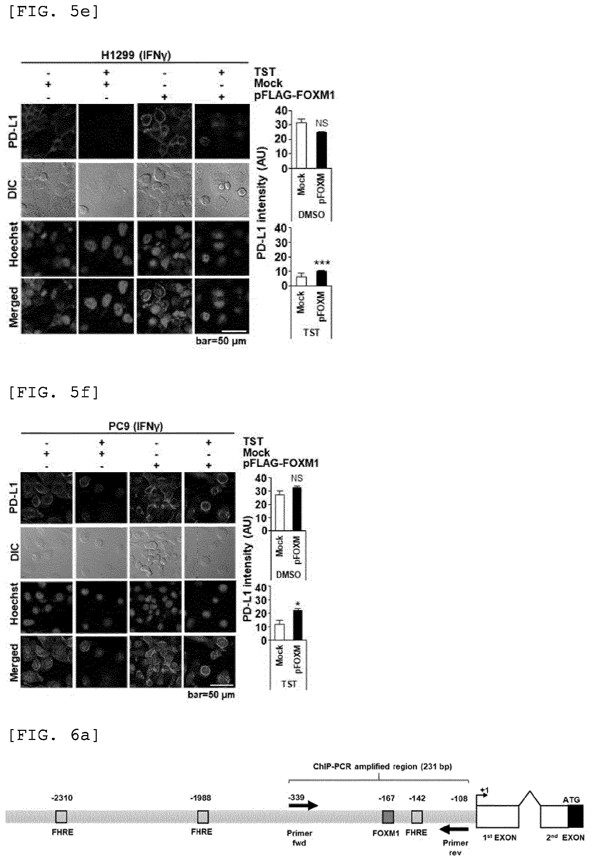

FIG. 5 shows the results confirming that the reduced expression of PD-L1 is restored by overexpression of FOXM1, as determined by immunoblot analysis (FIGS. 5a and 5b) or fluorescence imaging (FIGS. 5c to 5f).

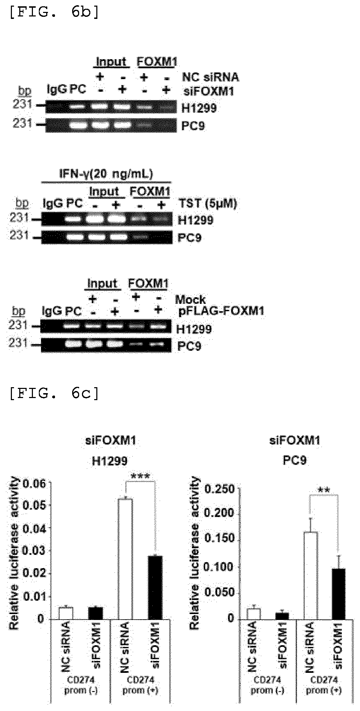

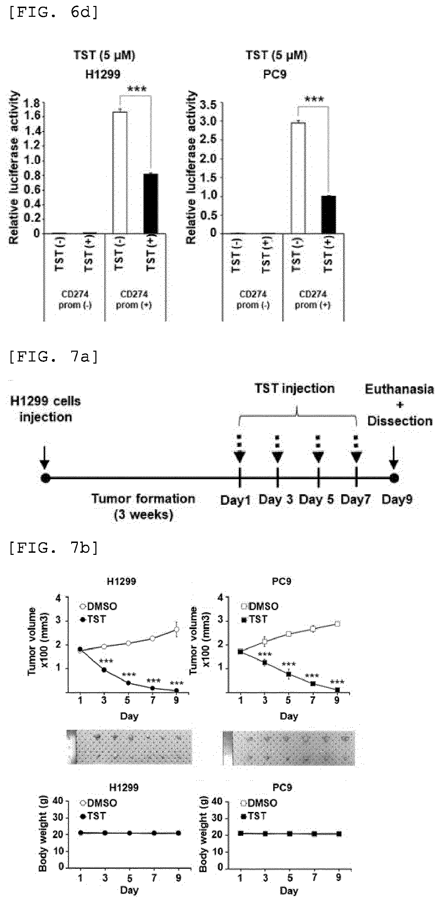

FIG. 6 shows the results confirming that FOXM1 binds to a promoter of the gene encoding PD-L1 and mediates the expression of PD-L1.

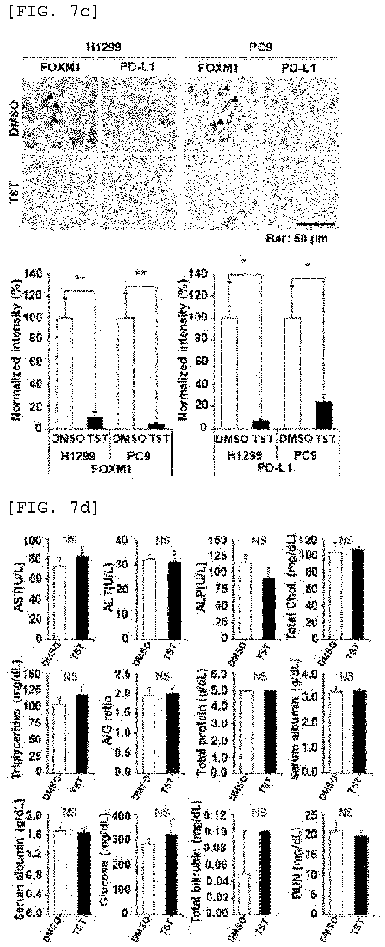

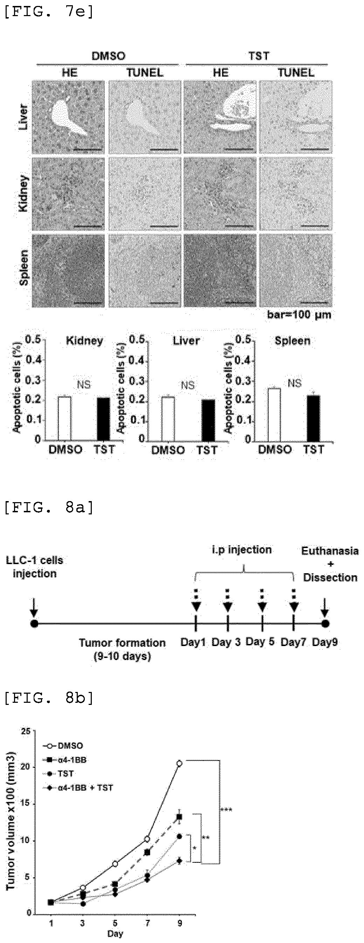

FIG. 7 shows the results confirming that thiostrepton (TST) inhibits tumor growth and expression of PD-L1 *in vivo.* FIGS. 7a to 7c show a timeline for tumor formation and thiostrepton administration performed on BALB/c mice, and the results confirming the tumor volume, weight, and expression level accordingly. FIG. 7d shows the results of analyzing the blood biochemical data in control mice and thiostrepton-treated mice. FIG. 7e shows the results of analyzing an apoptosis rate in these normal tissues by performing hematoxylin & eosin (H&E) staining and TUNEL staining on tissue sections of the liver, kidney, and spleen in control mice and thiostrepton-treated mice.

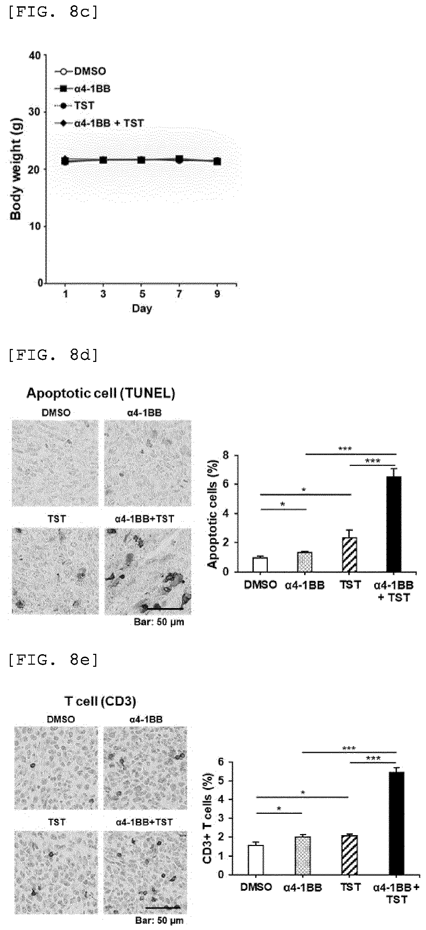

FIG. 8 shows the results confirming the effect of the combination of thiostrepton (TST) and anti-4-1BB antibody on antitumor in a syngeneic Lewis lung cancer (LLC-1) animal model. FIGS. 8a to 8c show the timelines for tumor formation and drug administration performed on C57BL/6 mice, and the results confirming tumor volume and body weight accordingly. FIGS. 8d and 8e show the results of performing terminal deoxynucleotidyl transferase dUTP nick end labeling (TUNEL) assays on mice in FIG. 8a to analyze apoptotic cell death, and confirming CD3⁺ T cell levels by immunohistochemistry.

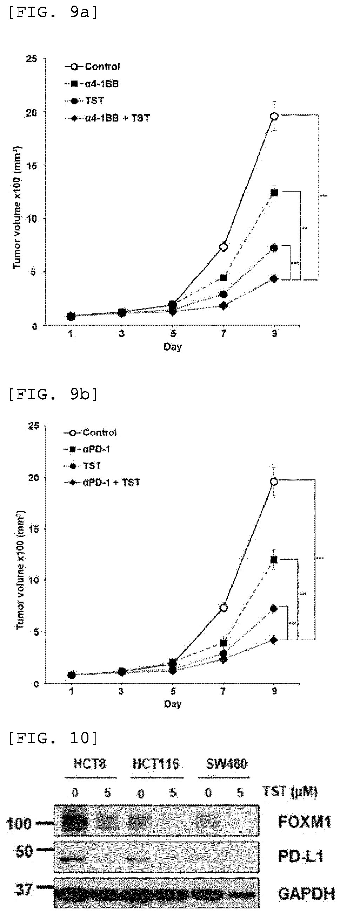

FIG. 9 shows the results confirming the synergistic effect on antitumor when combined with tyrostrepton (TST) and immune checkpoint inhibitors in a syngeneic Lewis lung cancer (LLC-1) animal model using C57BL/6 mice. FIG. 9a shows a timeline of tumor formation and drug administration in the animal model. FIG. 9b shows the results confirming the synergistic effect on antitumor when combined with thiostrepton (TST) and anti-4-1BB antibody compared to when each of them was treated alone. FIG. 9c shows the results confirming the synergistic effect on antitumor when combined with thiostrepton (TST) and anti-PD-1 antibody compared to when each of them was treated alone.
FIG. 10 shows the results confirming the expression of FOXM1 and PD-L1 in human colorectal cancer cell lines treated with thiostrepton (TST) by immunoblotting analysis.

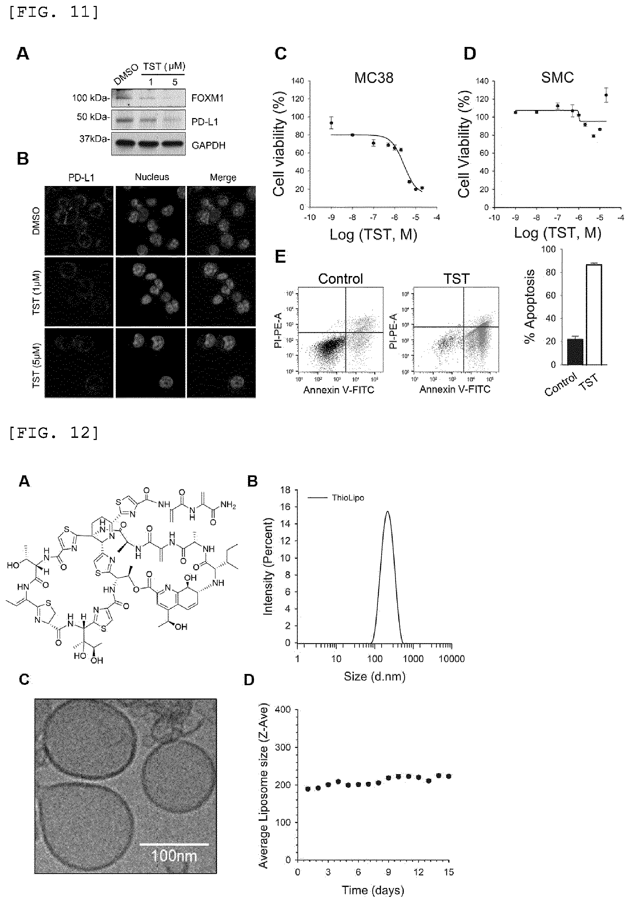

FIG. 11 shows the results confirming the effect of thiostrepton (TST) treatment on inhibiting PD-L1 expression and increasing cancer cell death in MC38 colorectal cancer cells. FIG. 11a shows the results confirming that the FOXM1 and PD-L1 expression decreases in a concentration-dependent manner when treated with thiostrepton (TST) by immunoblotting analysis. FIG. 11b shows the results confirming that PD-L1 expression on the surface of cancer cells decreases in a concentration-dependent manner when treated with thiostrepton (TST) by fluorescence image analysis. FIG. 11c shows data showing cell viability according to the concentration of thiostrepton (TST) treatment in MC38 cancer cells. FIG. 11d shows data showing cell viability according to the concentration of thiostrepton (TST) treatment in human primary coronary artery smooth muscle cells (SMCs), which are normal cells. FIG. 11e shows the results of analyzing the apoptosis rate of MC38 cells in the control group and the thiostrepton (TST)-treated group by a flow cytometer.
FIG. 12 shows the results confirming the characteristics of thiostrepton-loaded liposomes (ThioLipo). FIG. 12a shows a chemical structure of thiostrepton (TST), and FIGS. 12b and 12c show the results of measuring size distribution of ThioLipos in an aqueous solution and a transmission electron microscope (TEM) photograph. FIG. 12d shows the results of evaluating the stability of ThioLipos by measuring the change in size distribution over time for ThioLipos.

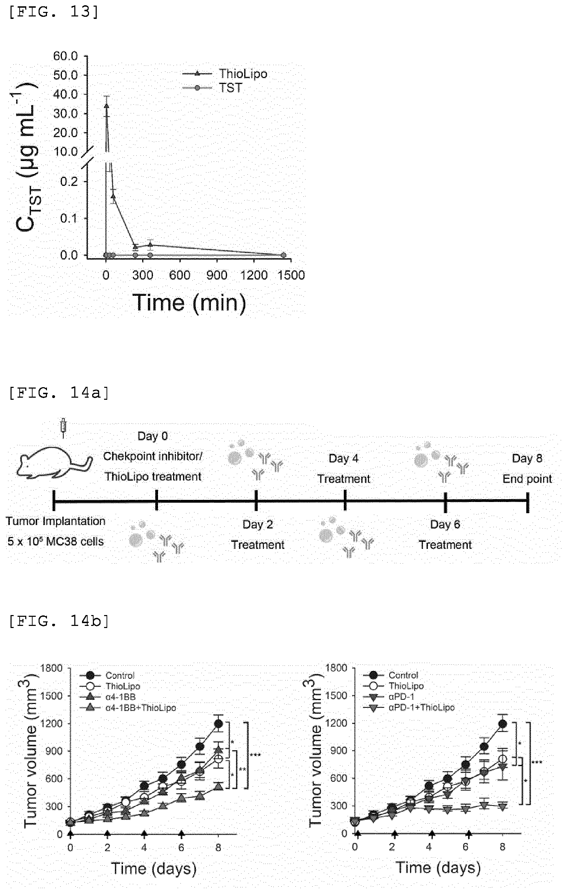

FIG. 13 shows the results of analyzing the change in blood concentration over time after intravenous administration of the same dose of free thiostrepton (TST) and ThioLipo.

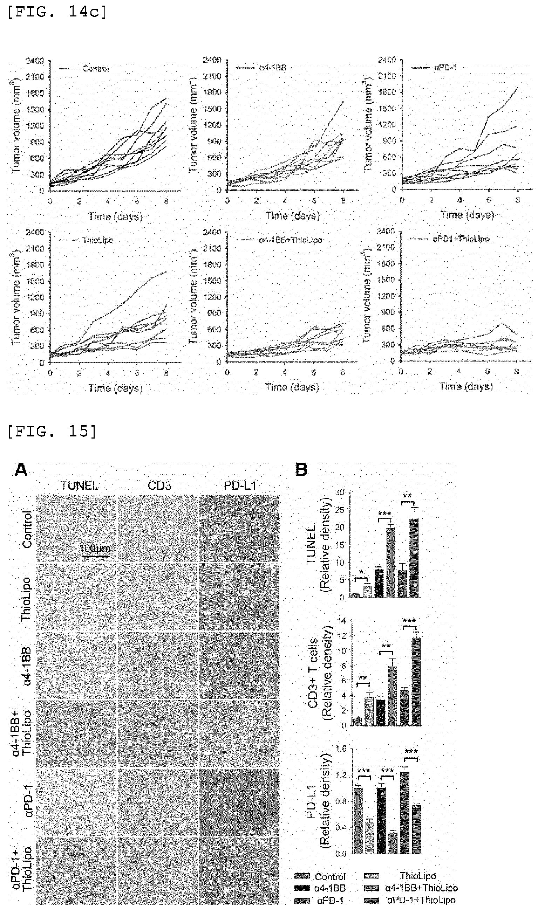

FIG. 14 shows the results confirming the synergistic antitumor effect by combined administration of ThioLipos and immune checkpoint inhibitors in a syngeneic colorectal cancer model. FIG. 14a shows a timeline for the administration of immune checkpoint inhibitors and ThioLipo performed on mice, and FIG. 14b shows the results of tumor growth curves when ThioLipo and anti-4-1BB antibodies were administered alone or in combination, or when ThioLipo and anti-PD-1 antibodies were administered alone or in combination to mice in FIG. 14a. FIG. 14c shows the results of tumor growth curves for individual mice for each treatment group in FIG. 14b.
FIG. 15 shows the results of performing tissue immunostaining on tumor sections of mice administered with ThioLipo and immune checkpoint inhibitors alone or in combination to confirm the expression of TUNEL, CD3, and PD-L1 and quantitatively analyzing the results.

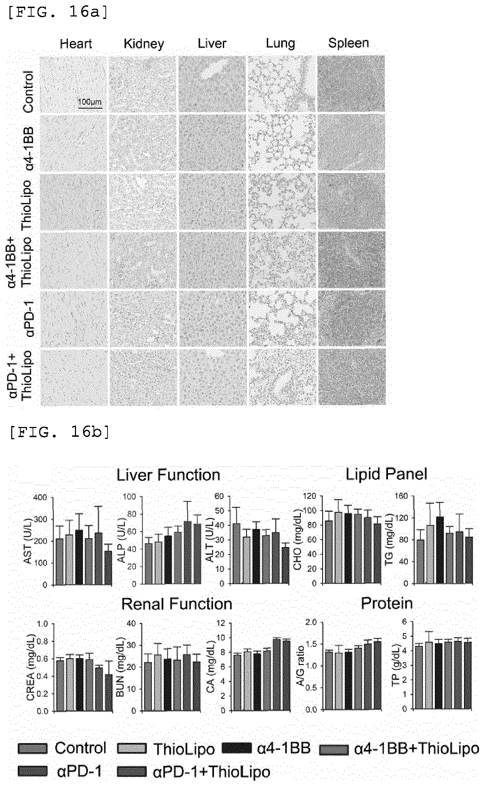

FIG. 16 shows that there were no side effects when ThioLipo and immune checkpoint inhibitors were administered alone or in combination. FIG. 16a shows the results of TUNEL staining performed on sections of major organs (heart, kidney, liver, lung, and spleen), and no histopathological changes or increased apoptotic cell death of normal cells were observed. FIG. 16b shows the results of analyzing blood biochemical data of mice, and no side effects were observed.

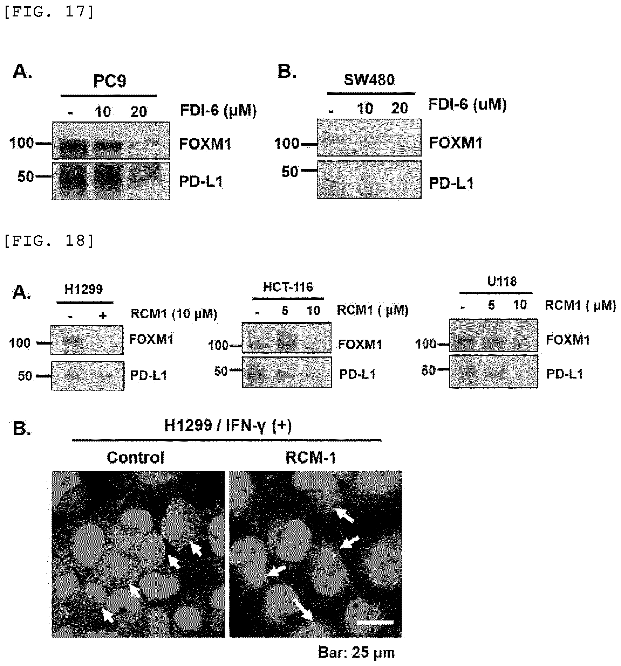

FIG. 17 shows the results confirming that FOXM1 and PD-L1 were downregulated by FDI-6 in lung adenocarcinoma (LUAD) and colorectal cancer cell lines. FIG. 17a shows the results confirming that both FOXM1 expression and PD-L1 expression decreased in proportion to the concentration of FDI-6 treated in lung cancer cell lines by immunoblotting. FIG. 17b is the results confirming that both FOXM1 expression and PD-L1 expression decreased in proportion to the concentration of FDI-6 treated in colorectal cancer cell lines by immunoblotting.
FIG. 18 shows the results confirming that the expression of FOXM1 and PD-L1 was downregulated by RCM1 treatment in lung cancer cells, colorectal cancer cells, and brain tumor cells. FIG. 18a shows the results confirming that the both FOXM1 expression and PD-L1 expression were inhibited in proportion to RCM1 treatment concentration in lung cancer, colorectal cancer, and brain tumor cell lines by immunoblotting. FIG. 18b is the results confirming that PD-L1 expression on the surface of cancer cells decreased when RCM1 is treated in human lung cancer cell lines by fluorescence imaging.

### [Best Mode]

Hereinafter, the present disclosure will be described in more detail with reference to the following Examples. However, these Examples are intended to illustrate the present disclosure by way of example, and the scope of the present disclosure is not limited to these Examples.

### Example 1: Confirmation of association of FOXM1 and PD-L1 expression

### 1.1. Information analysis of FOXM1 and PD-L1 in lung adenocarcinoma (LUAD)

### (1) Dataset for information analysis of FOXM1 and PD-L1 in lung adenocarcinoma (LUAD)

To determine the role of FOXM1 and PD-L1 in the prognosis of LUAD, the Cancer Genome Atlas (TCGA) LUAD Firehose Legacy (n = 586), which is available on cBioPortal for cancer genome open access resources, was used.

### (2) Geneset enrichment analysis (GEA)

DEGs were catalogued in response to siFOXM1 knockdown, and significantly downregulated genes (< 0.5-fold between NC siRNA and siFOXM1 knockdown H1299 and PC9 cells) were subjected to GSEA using the Enrichr database.

### 1.2. Cell culture and transfection

H1299 and PC9 cells purchased from ATCC (Manassas, VA, USA) were cultured in RPMI-1640 medium (#10-040-CV; Corning, New York, NY, USA) supplemented with 10% human serum albumin (#35-010-CV; Corning) and penicillin-streptomycin antibiotic cocktail (#15240062; Thermo Fisher Scientific, Waltham, MA, USA) at 37°C and 5% CO₂.

H1299 and PC9 LUAD cells were transfected with 5 nM negative control (NC) or FOXM1 siRNA (siFOXM1) (QIAGEN, Hilden, Germany) using Lipofectamine RNAiMAX (#13778150; Thermo Fisher Scientific). Human FOXM1 isoform b (pFLAG-FOXM1)-expressing plasmid or empty vector (pFLAG-CMV; mock) was transfected into H1299 and PC9 cells using Lipofectamine 2000 reagent (#11668019; Thermo Fisher Scientific) according to the manufacturer's instructions.

### 1.3. Reverse transcription and semi-quantitative PCR

Total RNA was extracted and purified using the RNeasy kit (#74004; QIAGEN) according to the manufacturer's protocol. Reverse transcription of mRNA was performed using a PrimeScript 1st Strand cDNA Synthesis Kit (#6110A; TaKaRa Bio, Otsu, Japan). Quantitative PCR was performed using primers specific for the CD274 and FOXM1 genes designed using Primer 3 software. A β-actin gene was used as an internal control group to normalize target gene expression. The primer pair sequences used in the present disclosure are listed in Table 1.

**[Table 1]**

| Primer classification | Classifi cation | Base sequence | SEQ ID NO: |
|---|---|---|---|
| < -actin | Forward | CAT GTT TGA GAC CTT CAA CAC CCC | 1 |
| | Reverse | GCC ATC TCC TGC TCG AAT TAT AG | 2 |
| FOXM1 | Forward | ATC TCA GCA CCA CTC CCT TG | 3 |
| | Reverse | CTT GCT GAG GCT GTC ATTCA | 4 |
| CD274 | Forward | TAT GGT GGT GCC GAC TAC AA | 5 |
| | Reverse | TGC TTG TCC AGA TGA CTT CG | 6 |
| CD274-Ch1P | Forward | CAA GGT GCG TTC AGA TGT TG | 7 |
| | Reverse | TCC TGA CCT TCG GTG AAA TC | 8 |

### 1.4. Confirmation of association of FOXM1 and PD-L1 expression in lung cancer cells

To investigate the clinical association of FOXM1 and PD-L1 in adenocarcinoma patients, survival curves of lung adenocarcinoma (LUAD) from the Cancer Genome Atlas (TCGA) dataset of cBioPortal were analyzed. The 70 patients with higher FOXM1 and PD-L1 expression had a shorter median overall survival rate than the 443 patients with no change in FOXM1 and PD-L1 expression (31.21 months vs. 50.20 months, p = 8.716 X 10⁻⁴, FIG. 1a).

In addition, to confirm whether FOXM1, which is a putative transcription factor (TF) regulating PD-L1 expression, is associated with the poor prognosis of LUAD patients, lung cancer tissues and corresponding normal tissues were immunostained with antibodies against FOXM1 and PD-L1 to compare the expression of FOXM1 and PD-L1. As a result, FOXM1 and PD-L1 were upregulated in LUAD tissues when compared to adjacent normal tissues (FIG. 1b).

RNA-seq analysis was performed on H1299 and PC9 cells subjected to FOXM1 knockdown by siRNA, and as a result, among 67 differentially expressed genes (DEGs) with a lower than 0.5-fold expression in both cell lines, the CD274 gene encoding PD-L1 was assumed to be a target of FOXM1 (FIG. 1c). To confirm the biological functions of downregulated genes after knockdown of FOXM1 in H1299 and PC9 cells, we subjected the downregulated genes (67 in total) to gene set enrichment analysis (GSEA) using the Enrichr database. Positive transcriptional regulation by RNA polymerase II and cell cycle regulation were significantly activated in the target genes (FIG. 1d). This suggests that FOXM1 may also contribute to CD274 regulation. The encoded PD-L1 protein plays an important role in immune evasion of lung cancer cells. After knockdown of FOXM1 with siRNA, PD-L1 expression was confirmed, and as a result, the PD-L1 transcript level was significantly decreased in both cell lines (FIG. 1e).

Additionally, after FOXM1 overexpression using pFLAG-FOXM1 isoform b, a major isoform expressed under physiological conditions, PD-L1 transcript levels were evaluated, and as a result, overexpression of FOXM1 significantly increased PD-L1 mRNA expression in H1299 and PC9 cells (FIG. 1f).

The potential effect of FOXM1 on PD-L1 expression in NSCLC cells was evaluated using FOXM1 inhibition strategies other than siRNA knockdown. Several FOXM1 inhibitors including TST, a natural product that inhibits FOXM1, were tested in H1299 and PC9 cells, of which TST was the most effective inhibitor, significantly reducing FOXM1 levels.

To induce an increase in PD-L1 expression, PD-L1 transcript levels were analyzed in cells treated with TST in the presence of IFN-γ, and as a result, TST-induced FOXM1 depletion caused a concentration-dependent decrease in PD-L1 mRNA expression in both the cell lines (FIG. 1g).

### Example 2: Confirmation of effects of FOXM1 Inhibition on cells

### 2.1. Cell analysis methods

### (1) Cell proliferation assay

H1299 and PC9 cells were grown in 96-well plates, treated with 5 µM TST, or transfected with siFOXM1 at a density of 3,000 cells/well in 6-well plates. Cell proliferation was recorded at the specified times by adding 4-[3-(4-iodophenyl)-2-(4-nitro-phenyl)-2H-5-tetrazolio]-1,3-benzene sulfonate) (WST-1, #MK400, TaKaRa Bio) at 37°C for 2 hours, and absorbance was measured at 450 nm. All experiments were performed in quadruplicate.

### (2) Cell cycle distribution analysis

Both H1299 and PC9 cells were enzymatically dissociated using TrypLE (#12605-010; Thermo Fisher Scientific), collected by centrifugation at 2,000 × g for 2 minutes, resuspended in PBS, fixed in 70% ethanol, and stored overnight at 4°C. Fixed cells were pelleted, washed twice with PBS, resuspended in FxCycle propidium iodide (PI)/RNase staining solution (#F10797; Thermo Fisher Scientific), and incubated in the dark at 37°C for 30 minutes. Flow cytometry was performed using a FACS-LSR Fortessa flow cytometer (BD Biosciences, Santa Clara, CA, USA).

### (3) Apoptotic cell death assay (Annexin V/PI staining)

H1299 and PC9 cells (1 x 10⁵) were cultured in 6-well plates, transfected with siFOXM1 or NC siRNA or treated with 5 µM TST or dimethyl sulfoxide (DMSO), and then stained using fluorescein isothiocyanate (FITC) Annexin V Apoptosis Detection Kit I (#556547; BD Biosciences). Cells were analyzed within 1 hour to reduce side effects due to staining, and flow cytometry was performed using a FACSVerse cell analyzer (BD Biosciences).

### 2.2. Inhibition of cell growth and induction of apoptotic cell death by FOXM1 Inhibition

To confirm the effect of FOXM1 on cell proliferation and survival in NSCLC, time-course experiments were performed for 24, 48, and 72 hours, and as a result, cell proliferation decreased in a time-dependent manner in both cell lines in response to siRNA-mediated knockdown and treatment with 5 µM TST (FIGS. 2a and 2b). In addition, the protein content using flow cytometry for cells treated with siFOXM1 or TST was analyzed, and as a result, FOXM1 knockdown by siFOXM1 or inhibition by TST increased the sub-G0/G1 population and decreased a S phase population in a time-dependent manner compared to the negative control (NC) siRNA or dimethyl sulfoxide (DMSO)-treated group (FIGS. 2c and 2d). Both cell lines treated with TST showed an increase in the G2 population with a decrease in the S phase population compared to the DMSO-treated group. This confirmed that FOXM1 depletion alters cell cycle progression in NSCLC cells and plays an important role in G1-S phase and G2-M phase transition. Furthermore, it was confirmed that the apoptotic cell population (annexin V/PI positive cells) significantly increased in a time-dependent manner in FOXM1-depleted cells by siRNA or TST treatment (FIGS. 2e and 2f).

The effect of reduced FOXM1 expression on c-MYC, cyclin B1, cyclin E1, and cyclin D1 in both cell lines through Western blot analysis was further confirmed, and as a result, the protein levels of c-MYC, cyclin B1, and cyclin D1 were significantly decreased after depletion of FOXM1 expression by siRNA or TST treatment (FIGS. 2g and 2h).

### 2.3. PD-L1 downregulation in lung cancer cell membranes due to FOXM1 depletion

Since PD-L1 is a transmembrane protein, and FOXM1 knockdown by siRNA or TST treatment reduces total PD-L1 protein levels, we performed immunocytochemistry (ICC) to evaluate whether this effect was phenotypically observed in the cell membrane. Membrane PD-L1 enrichment was also evaluated in H1299 and PC9 cells by ICC after transfection with siFOXM1 or NC siRNA for 72 hours, and as a result, PD-L1 membrane expression in H1299 control cells was significantly higher than in siFOXM1-transfected cells (FIG. 3a). FOXM1 downregulation resulted in a 67.4% reduction in PD-L1 expression (FIG. 3e). siFOXM1-transfected PC9 cells downregulated FOXM1 and this resulted in an 81.68% reduction in PD-L1 expression level (FIGS. 3b and 3f).

Considering that TST treatment in the presence of IFNγ decreased the total PD-L1 protein levels in lung cancer cells, we investigated the effect of TST on PD-L1 enrichment in the cell membrane, and as a result, IFNγ-boosted (IFNγ(+)) membrane PD-L1 levels were observed in both cell lines (FIGS. 3c and 3d). PD-L1 was decreased in the membranes of TST-treated H1299 and PC9 cells (FIGS. 3c and 3d). FOXM1 downregulation resulted in 87.23% and 85.82% reduction in the PD-L1 levels in H1299 and PC9 cells, respectively, compared to the DMSO control group (FIGS. 3e and 3f).

### Example 3: Confirmation of inhibitory effect of FOXM1 by TST

### 3.1. Protein Analysis

### (1) Nuclear cytoplasmic fractionation

To confirm protein subcellular localization, lysis of H1299 and PC9 cell pellets was performed in Buffer A (10 mM HEPES, 10 mM KCl, 1.5 mM MgCl₂, 0.5 mM dithiothreitol (DTT), and 0.05% IGEPAL CA-630 (pH 7.9)) for 10 minutes. The Buffer A lysate was centrifuged at 1,000 x g for 10 minutes at 4°C to collect the nuclear fraction. After removal of the cytosolic fraction, buffer B containing 5 mM HEPES, 0.2 mM EDTA, 1.5 mM MgCl₂, 0.5 mM DTT, and 26% glycerol (v/v) (pH 7.9) was added to the nuclear fraction and lysed on ice for 30 minutes.

### (2) Immunoblotting analysis of protein expression

Cells were lysed in RIPA buffer supplemented with complete mini protease inhibitor cocktail (#04693124001; Roche, Basel, Switzerland) and kept on ice for 30 minutes. Equal amounts of protein were evenly loaded and analyzed by sodium dodecyl sulfate-polyacrylamide gel electrophoresis (SDS-PAGE), and transferred to polyvinylidene difluoride (PVDF) membranes (#IPVH 00010; Millipore, Billerica, MA, USA) at 100 V for 1 hour. Primary antibodies (Santa Cruz Biotechnology, Texas, USA) specific for FOXM1 (#sc-271746), cyclin E1 (#sc-481), α-tubulin (#sc-8035), and β-actin (#sc-47778); PD-L1 (#13684S), cyclin D1 (#2978S), cyclin B1 (#4138S), c-MYC (#5605S), and poly(ADP-ribose) polymerase (PARP, #9542S; Cell Signaling Technology, Beverly, MA, USA) were used to probe proteins of interest. A horseradish-peroxidase-conjugated secondary antibodies specific to the primary antibody was used to detect proteins using pico-enhanced chemiluminescence substrate (#34577; Thermo Fisher Scientific).

### (3) Immunocytochemistry (ICC)

Cells were fixed in 4% paraformaldehyde (#30525-89-4; FUJIFILM Wako Chemical, Wako, Japan) for 15 minutes and blocked with 1% bovine serum albumin (BSA) in phosphate-buffered saline-0.01% Tween-20 (PBS-T) to reduce nonspecific antibody binding. Cells were then incubated overnight with an anti-PD-L1 antibody (#14598382; eBioscience, San Diego, CA, USA) diluted 1:200 and an anti-FOXM1 antibody (#sc-271746; Santa Cruz Biotechnology) diluted 1:50, followed by incubation with an Alexa Fluor 488-labeled secondary antibody (#A11029; Thermo Fisher Scientific) for 1 hour. Nuclei were stained with Hoechst 33342. After incubation, cells were washed with PBS and mounted on glass slides using Prolong Glass Antifade mounting medium (#P36982; Thermo Fisher Scientific). Confocal microscopy was performed using a Zeiss 730 Meta microscope (Carl Zeiss, Oberkochen, Germany) and analyzed using Axiovision software (Carl Zeiss).

### 3.2. Confirmation of effect of TST on inhibition of FOXM1 nuclear translocation

The nuclear translocation of FOXM1 has been highlighted as a prominent feature in various cancers. FOXM1 enrichment was examined in the nuclei of pFLAG-FOXM1-transfected H1299 and PC9 cells, followed by siFOXM1 knockdown or TST treatment (5 µM) in the presence of IFN-γ (20 ng mL⁻¹). Although FOXM1 was present in the nucleus and cytoplasm, overexpression of FOXM1 increased nuclear translocation of cytoplasmic FOXM1. siFOXM1-mediated FOXM1 inhibition or TST treatment decreased the protein levels and nuclear translocation of FOXM1 in H1299 and PC9 cells (FIGS. 4a and 4b). Compared to mock transfection, overexpression of FOXM1 restored FOXM1 levels by 48.85% and 84.33% for siFOXM1 and TST treatment in H1299 cells, respectively. The restoration values in PC9 cells were 30.95% and 57.56% for siFOXM1 and TST treatment, respectively. ICC analysis showed that FOXM1 was mainly localized in the nucleus of H1299 cells transfected with pFLAG-FOXM1 and PC9 cells transfected with NC siRNA, compared to siFOXM1-transfected cells (FIGS. 4c and 4d). Similarly, in H1299 cells and PC9 cells transfected with pFLAG-FOXM1, FOXM1 expression was mainly localized in the nucleus compared to TST-treated cells, where FOXM1 expression and nuclear translocation were inhibited. pFLAG-FOXM1-mediated FOXM1 overexpression was significantly decreased to 54.22% in H1299 cells (*p* < 0.01) and 35% in PC9 cells (*p* < 0.001) by siRNA-mediated knockdown. In the TST-treated cells, FOXM1 overexpression was significantly decreased by 63.7% in H1299 cells (p < 0.05) and 44% in PC9 cells (p < 0.01).

### Example 4: Elucidation of regulatory mechanism of PD-L1 expression by FOXM1

### 4.1. Confirmation of restoration of PD-L1 expression by FOXM1 overexpression

The restoration of PD-L1 expression was confirmed by inducing the overexpression of FOXM1 in FOXM1-knockdown cell lines. Specifically, H1299 and PC9 cells were transfected with NC siRNA or siFOXM1, followed by pFLAG-FOXM1 or an empty vector for 72 hours. As a result, Western blot analysis and confocal imaging of cells confirmed that siFOXM1-induced PD-L1 downregulation was restored in pFLAG-FOXM1-transfected cells, but not the in mock-transfected cells. FOXM1 allowed the restoration of PD-L1 expression on H1299 and PC9 cell membranes to 55.9% and 51.8%, respectively (FIGS. 5a, 5c, and 5d). Similarly, we investigated whether reduced PD-L1 expression could be restored in TST (5 µM)-treated H1299 and PC9 cells for 48 hours in the presence of IFNγ, and as a result, FOXM1 overexpression restored the reduced PD-L1 expression in FOXM1 knockdown cells, and TST treatment also restored the decreased PD-L1 expression in H1299 and PC9 cells. As shown by Western blotting (FIG. 5b) and confocal microscopy (FIGS. 5e and 5f), FOXM1 allowed PD-L1 expression restoration on the cell membrane of H1299 and PC9 cells by 38.7% and 46%, respectively. In addition, the elevated level of PD-L1 by pFLAG-FOXM1 overexpression (which mimics the situation in a malignant tumor mass) was significantly decreased to 52.6% in H1299 (p < 0.05) cells and 59.2% in PC9 cells (*p* < 0.01) by siRNA, and to 41.9% in H1299 cells (*p <* 0.01) and 68.0% in PC9 cells (*p* < 0.05) by TST treatment. Collectively, these results further support the hypothesis that PD-L1 is a downstream target of FOXM1 and is positively regulated by FOXM1, implying that PD-L1 levels can be finely regulated by inhibiting FOXM1.

### 4.2. Confirmation of PD-L1 expression regulation mechanism by FOXM1

### (1) Chromatin immunoprecipitation (ChIP)

To cross-link proteins to DNA, H1299 and PC9 cells were treated with 0.7% formaldehyde at 37°C for 10 minutes. Cross-linking was quenched by adding glycine to the culture medium at a final concentration of 0.125 M for 5 minutes. The cells were sonicated using a Bioruptor Next Gen sonicator (Diagenode, Denville, NJ, USA) for 5 minutes in 30 s on/30 s off cycle to obtain ~500 bp DNA fragments. The sonicated cell lysates containing genomic DNA fragments were pre-cleared to eliminate unwanted nonspecific components. ChIP was performed using antibody against FOXM1 (#sc-271746, Santa Cruz Biotechnology) with rotation at 4°C overnight, followed by various washing steps, reverse cross-linking, and protein digestion with proteinase K (#P2308; Sigma-Aldrich, St. Louis, MO, USA). The immunoprecipitated DNA fragments were purified using a phenol-chloroform method, and the purified DNA pellets were resuspended in TE buffer (pH 8.0) and used for PCR. Primers specifically detecting a FOXM1 binding region were designed using a Primer3 tool for chromatin immunoprecipitation-PCR (ChIP-PCR) (Table 1).

### (2) Dual-luciferase assay

The pGL3 plasmid containing a 2 kb promoter of CD274 (#107003; Addgene, Watertown, MA, USA) was kindly provided by Julian Downward lab (Coelho MA, de Carne Trecesson S, Rana S, Zecchin D, Moore C, Molina-Arcas M, East P, Spencer-Dene B, Nye E, Barnouin K, Snijders AP, Lai WS, Blackshear PJ, Downward J, Immunity 2017, 47, 1083.). H1299 and PC9 cells were co-transfected with a pGL3 2 kb promoter. Cells containing CD274 or control pGL3-basic plasmid and pGL4.70 hRluc Renilla luciferase vector (Promega, Madison, WI, USA) were transfected with siFOXM1 or treated with TST in the presence of interferon-gamma (IFNγ). Cells were harvested 48 hours after transfection, and a dual luciferase assay was performed according to the manufacturer's protocol (#E1910; Promega). At least four independent biological replicates were examined to obtain reliable results.

### (3) FOXM1 regulates expression of PD-L1 by binding directly to promoter

To elucidate the mechanism by which FOXM1 induces PD-L1 expression, ChIP-seq data from the ENCODE 3 database accessed from the UCSC Genome Browser (Human assembly Dec 2013, GRCh38/hg38) were analyzed. In K562 (a human immortalized myeloid leukemia cell line), FOXM1 enrichment was identified on a promoter region with a peak signal of approximately 167 bp upstream of a CD274 transcription start site, and the TAAAC FOXM1 consensus DNA-binding domain (DBD) was detected in two distant regions (-1,988 bp and -2,310 bp) as well as nearby (approximately 142 bp) from the CD274 transcription start site (FIG. 6a).

Based on these in silico data, we hypothesized that a CD274 promoter region in lung cancer cells is a potential target of the FOXM1 transcription factor. Thus, we performed a ChIP-PCR assay by excluding FOXM1-CD274 DNA complexes using an anti-FOXM1 antibody. The CD274 promoter region flanking putative FOXM1 binding sites (~167 nt) was detected by semiquantitative PCR in H1299 and PC9 cells, which was compared to a dramatic decrease in FOXM1 binding to the CD274 promoter observed in siFOXM1-transfected cells and TST-treated cells, along with their respective controls. The pFLAG-FOXM1-transfected cells showed increased binding of FOXM1 to the CD274 promoter compared to mock-transfected cells. These results indicate that FOXM1 directly binds to the CD274 promoter (FIG. 6b). Therefore, FOXM1 binds to the promoter and directly regulates PD-L1 transcription. Targeting FOXM1 disrupts FOXM1-mediated regulation of PD-L1 expression.

Additionally, we performed a dual-luciferase reporter assay using a CD274 promoter-luciferase construct to confirm whether depletion of FOXM1 binding by siRNA or TST treatment significantly reduces CD274 transcriptional activity. Consistent with the ChIP-PCR results, the depletion of FOXM1 by siRNA significantly decreased relative luciferase activity by 40% in H1299 cells (*p* < 0.001) and 42% in PC9 cells (*p* < 0.01) (FIG. 6c). Similarly, inhibition of FOXM1 by TST in the presence of IFNγ also significantly decreased CD274 promoter activity, as indicated by the decreased in relative luciferase activity by 53% in H1299 cells (*p* < 0.001) and 66% in PC9 cells (*p* < 0.001) (FIG. 6d).

These results support the conclusion that FOXM1 translocation to the nucleus positively regulates PD-L1 expression by directly binding to the CD274 promoter in both H1299 and PC9 cell lines. A novel model for FOXM1-mediated regulation of PD-L1 expression in lung cancer has been proposed. In this model, FOXM1 regulated PD-L1 expression by directly binding to the CD274 promoter, thereby inducing PD-L1 transactivation (FIG. 6g). Since FOXM1-induced PD-L1 upregulation results in immune evasion of lung tumor cells. Hence, targeting FOXM1 using TST, which is a natural FOXM1 inhibitor that appears to eliminate immune evasion and reduce tumor growth, may be a novel therapeutic strategy for PD-L1-mediated immune evasion in lung cancer cells.

### Example 5: Confirmation of effect of FOXM1 inhibition in vivo

### 5.1. Animal model

### (1) Xenograft tumor model

All animal studies were reviewed and approved by the Institutional Animal Care and Use Committee of the National Cancer Center Institute (NCC-21-619). Mice (n = 6) were injected intraperitoneally with TST dissolved in DMSO at a dose of 17 mg/kg every 2 days before euthanasia and sacrifice. Tumor volume and body weight of the mice were measured every other day. Tumor volume (V) was calculated as AB²/2 (where A and B are long and short diameters (mm) , respectively). Two days after the final TST injection, all mice were sacrificed, and tumor tissues were collected for immunohistochemical (IHC) staining of FOXM1 and PD-L1. The tissues were fixed in 10% neutralized formaldehyde and embedded in paraffin blocks. Tissue microarray (TMA) slides were made with a 4 mm diameter per sample and sectioned at 4 µm thickness to perform IHC with anti-FOXM1 (#20459S) or anti-PD-L1 (#13684S) (Cell Signaling Technology) antibodies. Autostainer Link48 (Dako, Agilent Technologies, Santa Clara, CA, USA) was used for analysis.

IHC staining was performed using anti-rabbit-HRP antibodies (K4003, EnVision+ HRP Labelled Polymer Anti-Rabbit, Dako, Agilent Technologies, Carpinteria, CA, USA) and 3,3'-diaminobenzidine (DAB) (K3468, DAB+ Chromogen, Dako, Agilent Technologies, Santa Clara, CA, USA) as substrates, and the sections were counterstained with hematoxylin (K8008, EnVision FLEX Hematoxylin, Dako, Agilent Technologies Singapore, Singapore). To analyze the *in vivo* toxicity of the tested drugs, four xenograft mice from each group were used for serum biochemical analysis (Table 2). On day 9, serum samples were collected from the mice in the control and TST-treated groups. In addition, the vital organs (liver, spleen, and kidney) were collected form mice, sectioned, and stained with H&E to monitor histopathological changes. Terminal deoxynucleotidyl transferase dUTP nick end labeling (TUNEL) assay (ApopTag Plus Proxidase In Situ kit, EMD Milipore, Temecula, CA, USA) was also performed on the sectioned tissues to analyze apoptotic cell death in response to the tested drug.

### (2) Syngeneic tumor model using murine lung cancer cells LLC-1

This animal studies were reviewed and approved by the Institutional Animal Care and Use Committee of the National Cancer Center Institute (NCC-22-675B). Lewis lung carcinoma LLC-1, murine lung cancer cells (5 x 10⁵), were implanted subcutaneously in 5-week-old C57BL/6N mice (n = 6 per group) (OrientBio, Seoul, Korea) and left for 10 days to form tumors with a volume of approximately 150 mm³. Then, mice were injected with TST at a dose of 17 mg/kg and anti-4-1BB antibody at a dose of 0.2 mg/mouse at the time points indicated by black arrows (day 1, day 3, day 5, and day 7). Tumor volumes and body weights of the mice were measured every other day. On day 9, all the mice were sacrificed, and tumor tissues were collected, fixed in formalin, embedded in paraffin, and sectioned for IHC. In addition, TUNEL assay was performed on the sectioned tissues to analyze apoptotic cell death in response to TST and anti-4-1BB antibody treatment.

Evaluation of CD3⁺ T-cells in tumor sections was performed as described in our previous paper (Huang Y, Lin C, Kao HK, Hung SY, Ko HJ, Huang YC, Chang YL, Chang KP, Cancer Manag Res 2020, 12,8275). CD3⁺ T-cells were detected by anti-CD3 antibody (ab16669, Abcam, Cambridge, UK) according to manufacturer's manuals. The positive signal intensity of CD3⁺ T-cells was analyzed by using computer image analysis with ImageJ software (LOCI, University of Wisconsin), and the intensity of CD3⁺ signals was calculated as a relative ratio of total positive area/total surface area. Pathological images were obtained from an Aperio VERSA slide scanning system (Leica Biosystems Imaging, Vista, CA, USA). At 100X magnification, three independent and intact computerized microscopic fields of each tissue sample were captured by Aperio Imagescope software (Leica Biosystems Imaging, Vista, CA, USA) and were used for analysis of signal intensity of CD3⁺ cells in while microscopic fields.

The *in vivo* synergy between TST and anti-4-1BB antibody-treated groups was determined by calculating a coefficient of drug interaction (CDI), with CDI values < 1, < 0.7, = 1, and > 1 indicating synergy, significant synergy, additivity, and antagonism, respectively.

In all statistical analyses, differences between groups were evaluated using the χ² test and Student's t-test. Statistical significance was set at *p* < 0.05, and deviation was expressed as the standard deviation.

### 5.2. Tumor growth inhibition and PD-L1 expression regulation by FOXM1 inhibition

To investigate the effect of TST, which is a FOXM1 inhibitor, on tumor growth and PD-L1 expression *in vivo,* human H1299 or PC9 cells (5 x 10⁶) were subcutaneously implanted into BALB/c nude mice (n = 6 per group). When the average size of the xenografted tumor masses reached approximately 170 mm³, TST was injected intraperitoneally into the mice at a dose of 17 mg/kg four times every 2 days (FIG. 7a). Regular monitoring of tumor volume during inhibitor treatment revealed a significant decrease in tumor volume in the TST-treated group compared to the control group. After 9 days, no difference in body weight was observed between the two groups (FIG. 7b). 2 days after the final TST treatment, the mice were sacrificed. Tumor tissues were collected, sectioned, and stained to analyze changes in the expression levels of FOXM1 and PD-L1. Immunohistochemical analysis demonstrated that FOXM1 and PD-L1 were overexpressed in lung tumors of control mice, but were significantly downregulated in TST-treated tumors (FIG. 7c). FOXM1 was mainly localized to the nuclei of control tumor cells, but showed faint immunopositivity in the nuclei of TST-treated tumor cells. These results indicated that PD-L1 was positively expressed in control tumors of H1299 and PC9 xenografts according to FOXM1 expression. In contrast, PD-L1 expression was significantly decreased by TST treatment (FIG. 7c).

Serum biochemical assay was performed to further evaluate the potential toxicity of TST *in vivo.* The levels of blood biomarkers were not significantly changed in the TST-treated groups compared to the DMSO control group (FIG. 7d and Table 2). Furthermore, hematoxylin and eosin (H&E) staining showed that no significant histological changes in vital organs were observed in the TST-treated group (FIG. 7e). TUNEL assay on tissue sections demonstrated that TST treatment has no effect on apoptotic cell death in normal tissues when compared to DMSO control tissues (FIG. 7e). Collectively, these results imply that TST-mediated FOXM1 inhibition can suppress immune evasion and effectively reduce tumor growth.

**[Table 2]**

| Blood biomarker classification | Control group (n = 4) | TST-treated group (n = 4) |
|---|---|---|
| ALT [U/L] | 32.00 ± 1.87 | 31.25 ± 4.21 |
| AST [U/L] | 72.00 ± 9.30 | 82.75 ± 8.58 |
| ALP [U/L] | 115.00 ± 10.42 | 91.50 ± 15.04 |
| Glu [mg/dL] | 281.75 ± 22.86 | 321.00 ± 58.73 |
| BUN [mg/dL] | 20.93 ± 2.87 | 19.70 ± 1.11 |
| Crea [mg/dL] | 0.03 ± 0.04 | 0.00 ± 0.00 |
| T-Bill [mg/dL] | 0.10 ± 0.05 | 0.10 ± 0.00 |
| T-chol [mg/dL] | 104.00 ± 10.70 | 107.50 ± 3.20 |
| TG [mg/dL] | 103.75 ± 9.07 | 118.00 ± 14.88 |
| TP [g/dL] | 4.93 ± 0.18 | 4.93 ± 0.08 |
| Alb [g/dL] | 3.25 ± 0.21 | 3.28 ± 0.08 |
| Glo [g/dL] | 1.68 ± 0.08 | 1.65 ± 0.09 |
| A/G ratio | 1.95 ± 0.19 | 1.99 ± 0.13 |

Abbreviations: ALT, alanine aminotransferase; AST, aspartate aminotransferase; ALP, alkaline phosphatase; Glu, glucose; BUN, blood urea nitrogen; Crea, creatinine; T-Bill, total bilirubin; T-chol, total cholesterol; TG, triglyceride; TP, total protein; Alb, serum albumin; Glo, serum globulin; A/G ratio, serum albumin/serum globulin ratio

### Example 6: Confirmation of synergistic antitumor effect by combined administration of FOXM1 inhibitor and immune checkpoint inhibitor

To evaluate the therapeutic potential of TST in cancer immunotherapy, an LLC-1 syngeneic tumor model, which has been reported to be resistant to ICI treatment, was used (FIG. 8a). Lewis lung carcinoma (LLC-1) mouse lung cancer cells (5 X 10⁵) were injected subcutaneously into C57BL/6N mice (n = 6 per group) and allowed to form tumors with a volume of 150 mm³ for 10 days. The mice were injected with TST at a dose of 17 mg/kg and anti-4-1BB antibody at a dose of 0.2 mg/mouse on day 1, day 3, day 5, and day 7. Treatment of tumor-bearing mice with anti-4-1BB antibody or TST alone resulted in 38% and 48% inhibition of tumor growth, respectively, compared to the control group (FIG. 8b). In particular, the combined treatment with anti-4-1BB antibody and TST showed a significantly enhanced therapeutic outcomes in tumor growth, recording a 65% inhibition of tumor growth in tumor-bearing mice compared to the control group (p *<* 0.001). No difference in body weight was observed between the experimental groups after 9 days (FIG. 8c).

Next, to investigate apoptotic cell death in the tumors, TUNEL staining of tumor sections was performed on day 9. The number of apoptotic tumor cells was 1.38-fold and 2.46-fold higher in an anti-4-1BB-treated group (p < 0.05) and TST-treated group (p < 0.05), respectively, than in the control group (FIG. 8d). The combination of TST with anti-4-1BB antibody synergistically increased the number of apoptotic LLC-1 tumor cells by 4.92-fold and 2.76-fold (CDI = 0.5) compared to the anti-4-1BB antibody-treated group (p < 0.001) or TST-treated group (p < 0.001), respectively. In addition, immunohistochemical staining for CD3 in tumor sections showed a 1.28-fold and 1.32-fold increase in CD3⁺ T cells in anti-4-1BB antibody-treated (p < 0.05) and TST-treated (p < 0.05) tumors, respectively, compared to the control group (FIG. 8d). The combination of TST with anti-4-1BB antibody also increased the CD3⁺ T-cell frequency in the tumor (CDI = 0.49) by 2.72-fold and 2.63-fold compared to the anti-4-1BB antibody-treated group (p < 0.001) or TST-treated group (p < 0.001), respectively (FIG. 8e). These results indicate that the combined treatment with TST and anti-4-1BB antibody has a significant synergistic effect on immunogenicity and antitumor activity. In particular, the combination of TST with anti-4-1BB antibody significantly delayed tumor growth and attracted T cells to the tumor area in an immune-resistant LLC1 lung tumor model. In addition, when the treatment was initiated at a smaller tumor size (i.e., ~80 mm³), the anti-4-1BB antibody and TST administration groups showed 36.6% and 63.1% tumor growth inhibition, respectively, and the TST and anti-4-1BB antibody combination administration group showed 78% tumor growth inhibition, indicating that the combined treatment could achieve a synergistic (CDI = 0.94) antitumor effect (FIG. 9a). In addition, the anti-PD-1 antibody (0.2 mg/mouse) and TST administration groups showed 38.7% and 63.1% tumor growth inhibition, respectively, and the TST and anti-PD-1 antibody combination administration group showed 78.4% tumor growth inhibition, indicating that a synergistic (CDI = 0.95) antitumor effect could be obtained through combined treatment (FIG. 9b). These results suggest that the application of TST, when combined with immune checkpoint inhibitors, is a clinically meaningful strategy to inhibit tumor growth and enhance the efficacy of immunotherapy.

### Example 7: Confirmation of effect of TST on colorectal cancer cell lines

### 7.1. Analysis of apoptotic cell death, viability, and protein expression

### (1) Reagents and cell culture used in experiment

Dimethyl sulfoxide (DMSO) and TST (Sigma-Aldrich, St. Louis, MO, USA), Amicon centrifugal filter device (MWCO 50 K, UFC805024, Merck Millipore), Sephadex G25 gel filtration column (PD-10; #17-0851-01, GE Healthcare), Anti-PD-1 (clone: RMP1-14, BP0146) and anti-4-1BB (clone: 17B5, BE0296) antibodies (BioXcell), Anti-CD3 antibody (ab16669, Abcam), Anti-PD-L1 antibody (13684, Cell Signaling Technology).

Human colorectal cancer cell lines (HCT116, HCT8, and SW480) and human primary coronary artery smooth muscle cells (SMCs) were purchased from the American Type Culture Collection (Manassas, VA, USA), and a MC38 mouse colorectal adenocarcinoma cell line was provided by Dr. Beom-Kyu Choi (National Cancer Center, Goyang, Republic of Korea). MC38 cells were cultured in Dulbecco's modified Eagle's medium (DMEM) supplemented with 10% FBS and 1% penicillin/streptomycin. SW480 and HCT8 cells were cultured in Roswell Park Memorial Institute (RPMI) medium supplemented with 10% FBS and 1% penicillin/streptomycin. HCT116 cells were maintained in McCoy's 5A medium supplemented with 10% FBS and 1% penicillin/streptomycin. Cells were maintained at 37°C in a humidified incubator with 5% CO₂.

### (2) Immunoblotting

For immunoblotting analysis, protein concentrations were measured using a bicinchoninic acid kit (Cat. 23225; Thermo Fisher Scientific, Carlsbad, CA, USA). Proteins were separated by SDS-PAGE electrophoresis. Primary antibodies against FOXM1 (#ab207298, Abcam), PD-L1 (#14-5982-82, Invitrogen), and glyceraldehyde-3-phosphate dehydrogenase (#sc-25778, Santa Cruz Biotechnology) were added and incubated overnight at 4°C. The appropriate secondary antibodies conjugated to horseradish peroxidase were bound to the washed membrane, and protein bands were detected using a chemiluminescence kit (Thermo Fisher Scientific, Carlsbad, CA, USA).

### (3) Apoptotic cell death assay (Annexin V assay)

MC38 cells (8.0 x 10⁴) were seeded in 6-well plates and cultured for 24 hours. The cells were treated with TST (5 µM) for 48 hours, harvested, and analyzed via FACS-Verse using a BD FITC Annexin V Apoptosis Detection Kit I (Cat No. 556547; BD Biosciences, San Diego, CA, USA) according to the manufacturer's protocol.

### (4) Cell viability assay

MC38 and SMC were cultured at a density of 3 x 10³ cells/well in 96-well plates. The cells were treated with various concentrations of TST dissolved in the medium for 48 hours, then WST-1 reagent (MK400; Takara) was added at a concentration of 10 µL/well and cultured for 1 hour. The absorbance was measured at 450 nm for further analysis.

### (5) Analysis of PD-L1 expression by TST treatment

To confirm the dose-dependent downregulation of PD-L1 levels by TST, which is a FOXM1 inhibitor, live cell imaging was performed on PD-L1 expression in MC38 cells. MC38 cells were cultured in Lab-Tek II and treated with various concentrations of TST (0, 1, 5, and 10 µM) for 48 hours. Afterwards, cells were incubated with anti-PD-L1 antibody (#14-5982-82; eBioscience, San Diego, CA, USA) and then conjugated with Alexa Fluor 594-labeled secondary antibody (A21209; Thermo Fisher, Waltham, MA, USA), and cell nuclei were stained with Hoechst33342 dye (Thermo Fisher, Waltham, MA, USA). Cells were washed three times with PBS (pH 7.4, 10 mM, 136 mM NaCl) and the medium was replaced with fresh complete medium. Fluorescence images of nuclei (λₑₓ 359 nm, λₑₘ 457 nm) and PD-L1 (λₑₓ 590 nm, λₑₘ 618 nm) were obtained using a confocal scanning laser microscope (LSM780; Carl Zeiss, Oberkochen, Germany).

### 7.2. Confirmation of effect of TST on PD-L1 expression in colorectal cancer cells

Analysis of the effect of TST on PD-L1 expression in human and mouse colorectal cancer cells showed that treatment with 5 µM TST significantly decreased both FOXM1 and PD-L1 levels in human colorectal cancer cells (HCT8, HCT116, and SW480) (FIG. 10). After treatment with TST, FOXM1 and PD-L1 expression levels were also dose-dependently decreased in MC38 mouse colorectal cancer cells, (FIG. 11a). Confocal fluorescence images for immunocytochemical analysis of PD-L1 expression showed a strong fluorescence signal on the surface of cancer cells (FIG. 11b). The level of PD-L1 in the plasma membrane was dose-dependently decreased after TST treatment. Similar to FOXM1 directly binds to the PD-L1 promoter in the nucleus and selectively upregulates PD-L1 expression, TST clearly decreased the expression of PD-L1 in human and mouse colon cancer cells due to the inhibition of FOXM1 by TST.

The *in vitro* cytotoxicity of TST on MC38 cancer cells and SMC normal cells was tested. The median inhibitory concentration (IC₅₀) of TST was 3.8 µM in MC38 cancer cells, but in contrast, no significant apoptotic cell death was observed in SMC normal cells. Flow cytometry showed that TST treatment significantly increased apoptotic cell death in MC38 cancer cells (FIG. 11e).

### Example 8: Confirmation of antitumor effect of ThioLipo

### 8.1. Preparation and characterization of ThioLipos

### (1) Preparation method of ThioLipos

Cholesterol, 1,2-dioleoyl-sn-glycero-3-phosphocholine (DOPC), and 1,2-disteroyl-sn-glycero-3-phosphoethanolamine-N-[methoxy(polyethylene glycol)-2000] (DSPE-PEG2000) were purchased from Avanti Polar Lipids (Alabama, USA). DOPC, cholesterol, and DSPE-PEG2000 were added to a round-bottom flask at molar ratios of 55, 40, and 5, respectively. TST was dissolved in chloroform at a concentration of 1.5 mg/mL, and 1 mL of the solution was added to the lipid sample. The solution was dried using a rotary evaporator to leave a thin lipid film. PBS (1 mL) was added to a round-bottom flask and sonicated in a bath sonicator at 25°C for 5 minutes. After removing the lipids from the flask, the solution was transferred to a dram vial and sonicated for 20 minutes using a probe sonicator. Uncaptured TST and other impurities were removed by centrifugation at 600 ×g for 5 minutes at 25°C. Free TST was further removed using a PD-10 size exclusion column. The size distribution (Z-average) and polydispersity index were characterized using dynamic light scattering (DLS; Malvern Zetasizer). The concentration of TST encapsulated in nanoliposomes was analyzed by dissolving the nanoliposomes in DMSO and measuring the absorbance at 300 nm using a UV-Vis spectrophotometer (DU730, Beckman Coulter, Brea, CA). For transmission electron microscopy (TEM) of ThioLipos, a grid (Quantifoil, R1.2/1.3, 200 mesh, EMS) was prepared with a hydrophilic surface using a glow discharge system (PELCO easiGlow, Ted pella), 4 µL of sample was added to the grid, and blotted for 1.5 s at 100% humidity and 4°C temperature, respectively. The samples were then quenched-frozen in liquid ethane for vitrification using a Vitrobot Mark IV (FEI). The samples were analyzed using a Talos L120C (FEI) at 120 kV at the NanoBioimaging Center (Seoul National University, Korea).

### (2) Characterization of ThioLipos

TST is a hydrophobic compound with low solubility in water, and liposome encapsulation has been adopted as a system to effectively deliver these hydrophobic drugs to tumors (FIG. 12a). The hydrodynamic size of the prepared ThioLipos was 192 nm, and the TEM image of ThioLipo showed a round-shaped monolayer structure (FIG. 12c). The stability of the ThioLipos was also tested. There was no significant change in the size distribution over 15 days (FIG. 12d). The encapsulation efficiency of TST in nanoliposomes was 63 ± 3%, and a TST to lipid ratio in nanoliposomes was 0.37 ± 0.018.

In addition, we investigated whether the blood concentration of TST in the form of ThioLipos could be enhanced in vivo. Due to the hydrophobic nature of TST, free TST was injected intraperitoneally and LipoThio was injected intravenously. Since a sensitive method for detecting TST in blood and other tissues has not been established, the TST concentration was analyzed using TripleTOF5600 with ultrahigh-performance liquid chromatography. Mice treated with ThioLipo showed significantly higher blood TST concentrations after 24 hours (FIG. 13).

### 8.2. Confirmation of effect of ThioLipo

### (1) syngeneic colorectal cancer model

All animal experiments were approved by the Institutional Animal Care and Use Committee of the National Cancer Center (NCC-21-615). MC38 cells (5 × 10⁵ cells) were injected subcutaneously into the right flank of C57BL/6 mice (6 weeks old; Orient Bio, Korea). When the tumor size reached approximately 150 mm³, mice were randomly assigned to treatment groups. Mice were treated with anti-4-1BB (10 mg/kg, n = 10) and anti-PD-1 (10 mg/kg, n = 9) antibodies via intraperitoneal injection on day 0 and every 2 days. ThioLipo was administered intravenously via tail vein at a dose of 10 mg TST equiv./kg on day 0 and every 2 days (n = 11). Mice in the anti-4-1BB+ThioLipo group (n = 10) were treated with anti-4-1BB antibody (10 mg/kg, intraperitoneal injection) and Thio-Lipo (10 mg TST equiv./kg, intravenous injection) on day 0 and every 2 days, while mice in the anti-PD-1+ThioLipo group (n = 8) were treated with anti-PD-1 antibody (10 mg/kg, intraperitoneal injection) and Thio-Lipo (10 mg TST equiv./kg, intravenous injection) on day 0 and every 2 days. Control mice (n = 9) were injected intravenously with PBS solution (100 µL) on day 0 and every 2 days. Mice were treated with antibodies and ThioLipo for a total of 4 times, and the tumor volumes and body weight of mice were measured daily until day 8, and the tumor volume (V) was calculated as AB²/2 (where A and B are the long and short diameters (mm), respectively). The synergistic antitumor effect of combined administration was determined by calculating a coefficient of drug interaction (CDI). The CDI was calculated using the following formula: CDI = AB/(A × B), where AB is a ratio of tumor size in the combined group compared to the control group, and A and B are a ratio of tumor size in the individual treatment groups compared to the control group. CDI values of < 1, < 0.7, = 1, and > 1 indicate synergy, significant synergy, additivity, and antagonism, respectively.

On day 8, mice were euthanized, and tumor tissues and normal vital organs (heart, lung, kidney, spleen, and liver) were collected, and fixed in 4% formaldehyde solution for immunohistochemical (IHC) and histopathological analysis. Tissues were paraffin-embedded, sectioned, and stained. IHC analysis for CD3 (1:150, ab16669; Abcam) and PD-L1 (1:100, #13684; Cell Signaling Technology) levels in tumor tissues was performed on paraffin-embedded sections using Ventana Roche Discovery XT Immunostainer (Mannheim, Germany) according to standard procedures for DAB-ChromoMap Discovery Studies. Antigen retrieval was initiated by heat-induced unmarking of epitopes while slides were immersed according to the manufacturer's instructions. Sections were incubated with primary antibodies at 37°C, followed by secondary antibodies application at room temperature. Detection was performed using a DAB ChromoMap detection kit according to a copper-enhanced diaminobenzidine development method, and then light counterstaining with hematoxylin was performed for 4 minutes. The sections were then manually dehydrated, cleared in xylene, and mounted on coverslips. In addition, an erminal deoxynucleotidyl transferase dUTP nick-end labeling (TUNEL) assay was performed to evaluate apoptotic cell death in the treatment group. Tissue sections were stained with hematoxylin and eosin (H&E) for histopathological analysis, and imaging of the stained tissue sections was performed using the Vectra Polaris system (Akoya Biosciences). Tissue sections were stained using a TUNEL assay kit to analyze apoptotic cell death in normal tissues of the treatment group and compared to the control group.

### (2) Serum biochemical analysis

To analyze the potential toxicity of each treatment regimen, serum was collected from mice in each group on the last day of the experiment. Albumin (ALB), creatinine (CREA), total protein (TP), blood urea nitrogen (BUN), glucose (GLU), aspartate transaminase (AST), alanine transaminase (ALT), alkaline phosphatase (ALP), creatine phosphokinase (CPK), total bilirubin (T-Bil), triglyceride (TG), phosphate (PHOS), calcium (CA), blood cholesterol (CHO) levels, and albumin/globulin ratio (A/G) were measured by TNPbio (Gyeonggi, Gwangju, Korea).

In all statistical analyses, differences between groups were evaluated using Student's t-test. Statistical significance was set at P < 0.05, and data were expressed as mean ± standard error of the mean.

### (3) Confirmation of efficacy of combined administration of immune checkpoint inhibitors and ThioLipos

In a syngeneic colorectal cancer model, treatment was initiated on day 0 when the tumor size reached 150 mm³ (FIG. 14a). ThioLipos was administered intravenously and antibody treatment was administered intraperitoneally. Each treatment group was treated with ThioLipo (10 mg of TST equiv./kg), anti-4-1BB antibody (10 mg/kg), or anti-PD-1 antibody (10 mg/kg). Treatment with anti-4-1BB antibody, anti-PD-1 antibody, or ThioLipo alone reduced tumor volume by 24% (p < 0.05), 39% (p < 0.05), and 31% *(p* < 0.05), respectively, compared to the control group (FIGS. 14b and 14c).

Although better therapeutic outcomes can be expected with single ICI treatment in small tumor size (80 mm³), we initiated experiment was at a tumor size of 150 mm³ to confirm the effect of combination therapy with ICI and ThioLipos in a rapidly growing tumor model. In particular, the combination therapy with anti-4-1BB+ThioLipo and anti-PD-1+ThioLipo groups showed synergistic antitumor effects, resulting in 56% (p < 0.001) and 74% (p < 0.001) tumor growth inhibition in tumor-bearing mice compared to the control group, respectively. The calculated CDI values for the anti-4-1BB+ThioLipo group and the anti-PD-1+ThioLipo group were 0.83 and 0.61, respectively, and no difference in body weight was observed between the treatment groups.

The enhanced therapeutic effect of ThioLipos was additionally analyzed by staining tumor sections using a TUNEL assay kit and IHC on day 8 (FIG. 15). Apoptotic cell death in tumors was increased by 3.2-fold, 8.1-fold, and 7.7-fold in the ThioLipo-treated group (P < 0.05), anti-4-1BB-treated group (p < 0.001), and anti-PD-1-treated group, respectively, compared to the control group. Apoptotic cell death in the anti-4-1BB+ThioLipo and anti-PD-1+ThioLipo groups was increased by 2.4-fold and 2.7-fold, respectively, compared to the anti-4-1BB-treated group (p < 0.001) and anti-PD-1-treated group (p < 0.01). IHC staining of tumor sections showed that CD3⁺ T cells were increased by 3.8-fold, 3.4-fold, and 4.7-fold in the ThioLipo-treated group (p *<* 0.01), anti-4-1BB-treated group (p < 0.001), and anti-PD-1-treated group (p < 0.001), respectively, compared with the control group. Tumor-infiltrating CD3⁺ T cells in the anti-4-1BB+ThioLipo and anti-PD-1+ThioLipo groups were increased by 2.3-fold and 3.4-fold, respectively, compared to the anti-4-1BB-treated group (P < 0.01) and anti-PD-1-treated group *(P <* 0.001). In addition, IHC staining for PD-L1 protein in tumor sections was performed to measure changes in PD-L1 levels. As expected, PD-L1 levels in tumor sections were significantly downregulated in ThioLipo-treated tumors (47.7% inhibition vs. control group, p < 0.001). PD-L1 levels in the combined administration group were also significantly lower than those in the ICI-treated group. Although the expression of PD-L1 was increased with anti-PD-1 treatment, the combined treatment with anti-PD-1 antibody and ThioLipos significantly inhibited PD-L1 levels in tumor tissues.

To confirm the side effects of the treatment regimen on the normal tissues of each treatment group *in vivo,* i.e., the side effects of the treatment regimen, the vital organs (heart, spleen, lung, kidney, and liver) were collected and sectioned, and H&E and TUNEL staining were performed on the tissue sections at the end of the experiment, and blood samples from the mice were also collected and analyzed. As described above, no difference in body weight was observed between the treatment groups. The TUNEL staining images of normal tissue sections did not show an increase in apoptotic cell death in any of the treatment group compared to the control group (FIG. 16a). Furthermore, no histopathological changes were observed in H&E staining images of the normal tissue sections. Serum biochemical analysis also showed no signs of toxic effects in any of the treatment group compared to the control group (FIG. 16b).

### Example 9: Confirmation of effects of FDI-6 and RCM1 on lung cancer, colorectal cancer, and brain tumor cell lines

### 9.1. Apoptotic cell death, viability, and protein expression analysis

### (1) Reagents and cell culture used in experiment

Dimethyl sulfoxide (DMSO), FDI-6 (Sigma-Aldrich, St. Louis, MO, USA, CAS Number: 313380-27-7), RCM1 (R&D Systems, Minneapolis, MN 55413, USA, CAS No: 339163-65-4), Amicon centrifugal filter device (MWCO 50 K, UFC805024, Merck Millipore), Sephadex G25 gel filtration column (PD-10; #17-0851-01, GE Healthcare), anti-PD-1 (clone: RMP1-14, BP0146), anti-4-1BB (clone: 17B5, BE0296) antibody (BioXcell), anti-CD3 Antibody (ab16669, Abcam), anti-PD-L1 antibody (13684, Cell Signaling Technology).

Human lung cancer cell lines (H1299, PC9), colorectal cancer cell lines (HCT116, SW480), and brain tumor cell lines (U118) were purchased from the American Type Culture Collection (Manassas, VA, USA). H1299, PC9, U118, and SW480 cells were cultured in Roswell Park Memorial Institute (RPMI) medium supplemented with 10% FBS and 1% penicillin/streptomycin, and HCT116 cells were cultured in McCoy's 5A medium supplemented with 10% FBS and 1% penicillin/streptomycin. Cells were maintained at 37°C in a humidified incubator with 5% CO₂.

### (2) Immunoblotting

For immunoblotting analysis, protein concentration was measured using a bicinchoninic acid kit (Cat. 23225; Thermo Fisher Scientific, Carlsbad, CA, USA), and proteins were separated by SDS-PAGE electrophoresis. Primary antibodies against FOXM1 (#ab207298, Abcam), PD-L1 (#14-5982-82, Invitrogen), and glyceraldehyde-3-phosphate dehydrogenase (#sc-25778, Santa Cruz Biotechnology) were added and incubated overnight at 4°C. After washing, appropriate secondary antibodies conjugated to horseradish peroxidase were bound to the membrane, and protein bands were detected using a chemiluminescence kit (Thermo Fisher Scientific, Carlsbad, CA, USA).

### (3) Analysis of PD-L1 expression in lung cancer cell lines by RCM1 treatment

To confirm the dose-dependent downregulation of PD-L1 expression by RCM1, which is a FOXM1 inhibitor, live cell imaging was performed on PD-L1 expression in H1299 cells, which is a lung cancer cell line. H1299 cells were cultured in Lab-Tek II and treated with RCM1 (10 µM) for 48 hours. Afterwards, cells were incubated with anti-PD-L1 antibody (#14-5982-82; eBioscience, San Diego, CA, USA), and then conjugation with Alexa Fluor 594-labeled secondary antibody (A21209; Thermo Fisher, Waltham, MA, USA), and cell nuclei were stained with Hoechst33342 dye (Thermo Fisher, Waltham, MA, USA). Cells were washed three times with PBS (pH 7.4, 10 mM, 136 mM NaCl), and the medium was replaced with fresh complete medium. Fluorescence images of nuclei (λₑₓ 359 nm, λₑₘ 457 nm) and PD-L1 (λₑₓ 590 nm, λₑₘ 618 nm) were obtained using a confocal scanning laser microscope (LSM780; Carl Zeiss, Oberkochen, Germany).

### 9.2. Confirmation of effect of FDI-6 and RCM1 on PD-L1 expression in lung cancer, colorectal cancer, and brain tumor cells

Analysis of the effect of FDI-6 and RCM1 on PD-L1 expression in lung cancer, colorectal cancer, and brain tumor cell lines showed that treatment with FDI-6 significantly decreased both FOXM1 and PD-L1 expression in a lung cancer cell line (PC9) and a colorectal cancer cell line (SW480) (FIGS 17a and 17b). Treatment with RCM1 also significantly decreased FOXM1 and PD-L1 expression in a lung cancer cell line (H1299), colorectal cancer cell line (HCT116), and brain tumor cell line (U118) (FIG. 18a). The results of confocal fluorescence imaging analysis also confirmed that the level of PD-L1 expressed on the surface of cancer cells was decreased after RCM1 treatment (FIG. 18b). Similar to FOXM1 directly binds to the PD-L1 promoter in the nucleus and selectively upregulates PD-L1 expression, FDI-6 and RCM1, which are FOXM1 inhibitors, were confirmed to decrease the expression of PD-L1 by suppressing FOXM1 in human lung cancer, colorectal cancer, and brain tumor cells.

From the above description, those skilled in the art to which the present disclosure pertains will understand that the present disclosure can be implemented in other specific forms without changing its technical idea or essential features. In this regard, it should be understood that the embodiments described above are exemplary in all respects and are not intended to be limiting. The scope of the present disclosure should be interpreted that all changes or modifications derived from the meaning and scope of patent claims to be described below rather than the detailed description above and their equivalent concepts are included in the scope of the present disclosure.

## Claims

1. A pharmaceutical composition for preventing or treating cancer, comprising a FOXM1 inhibitor and an immune checkpoint inhibitor as active ingredients.

2. The pharmaceutical composition of claim 1, wherein the FOXM1 inhibitor is at least one selected from the group consisting of thiazolidinedione, diarylheptanoid, RCM-1, thiostrepton, honokiol, FDI-6, Siomycin A, monensin, FOXM1 apt, and peptide 9R-P201.

3. The pharmaceutical composition of claim 1, wherein the immune checkpoint inhibitor is at least one selected from the group consisting of an anti-PD-L1 antibody, an anti-PD-1 antibody, an anti-CTLA4 antibody, an anti-PD-L2 antibody, an LTF2 regulatory antibody, an anti-LAG3 antibody, an anti-A2aR antibody, an anti-TIGIT antibody, an anti-TIM-3 antibody, an anti-B7-H3 antibody, an anti-B7-H4 antibody, an anti-VISTA antibody, an anti-CD47 antibody, an anti-BTLA antibody, an anti-KIR antibody, an anti-IDO antibody, and an anti-4-1BB antibody.

4. The pharmaceutical composition of claim 1, wherein the pharmaceutical composition reduces an expression of PD-L1.

5. The pharmaceutical composition of claim 1, wherein the FOXM1 inhibitor is loaded into a liposome.

6. The pharmaceutical composition of claim 1, wherein the pharmaceutical composition is for combined administration of a FOXM1 inhibitor and an immune checkpoint inhibitor.

7. The pharmaceutical composition of claim 6, wherein the pharmaceutical composition is in the form of a mixture of a FOXM1 inhibitor and an immune checkpoint inhibitor, or is formulated with each of the FOXM1 inhibitor and the immune checkpoint inhibitor and administered simultaneously or sequentially.

8. The pharmaceutical composition of claim 1, wherein the cancer is lung cancer, pancreatic cancer, pharyngeal cancer, laryngeal cancer, esophageal cancer, stomach cancer, duodenal cancer, small intestine cancer, colorectal cancer, colon cancer, rectal cancer, breast cancer, ovarian cancer, prostate cancer, liver cancer, thyroid cancer, kidney cancer, uterine cancer, brain tumor, skin cancer, melanoma, malignant bone tumor, bladder cancer, or blood cancer.

9. An anticancer adjuvant comprising a FOXM1 inhibitor and an immune checkpoint inhibitor as active ingredients.

10. The anticancer adjuvant of claim 9, wherein the FOXM1 inhibitor is at least one selected from the group consisting of thiazolidinedione, diarylheptanoid, RCM-1, thiostrepton, honokiol, FDI-6, Siomycin A, monensin, FOXM1 apt, and peptide 9R-P201.

11. The anticancer adjuvant of claim 9, wherein the immune checkpoint inhibitor is at least one selected from the group consisting of an anti-PD-L1 antibody, an anti-PD-1 antibody, an anti-CTLA4 antibody, an anti-PD-L2 antibody, an LTF2 regulatory antibody, an anti-LAG3 antibody, an anti-A2aR antibody, an anti-TIGIT antibody, an anti-TIM-3 antibody, an anti-B7-H3 antibody, an anti-B7-H4 antibody, an anti-VISTA antibody, an anti-CD47 antibody, an anti-BTLA antibody, an anti-KIR antibody, an anti-IDO antibody, and an anti-4-1BB antibody.

12. Use of a composition comprising a FOXM1 inhibitor and an immune checkpoint inhibitor for the prevention or treatment of cancer.

13. Use of a composition comprising a FOXM1 inhibitor and an immune checkpoint inhibitor for the manufacture of a medicament for the prevention or treatment of cancer.

14. A method for preventing or treating cancer, comprising administering to a subject a composition comprising a FOXM1 inhibitor and an immune checkpoint inhibitor.
